(19) **Europäisches Patentamt**
**European Patent Office**
**Office européen des brevets**

(11) **EP 2 048 158 B1**

(12) **EUROPEAN PATENT SPECIFICATION**

(45) Date of publication and mention of the grant of the patent:
**02.10.2013 Bulletin 2013/40**

(51) Int Cl.:
*C07K 14/56* $^{(2006.01)}$    *C12N 15/21* $^{(2006.01)}$
*A61K 38/21* $^{(2006.01)}$    *A61P 31/12* $^{(2006.01)}$
*A61P 35/00* $^{(2006.01)}$

(21) Application number: **07788693.5**

(22) Date of filing: **15.06.2007**

(86) International application number:
**PCT/ES2007/070117**

(87) International publication number:
**WO 2008/000881 (03.01.2008 Gazette 2008/01)**

(54) **GLYCOSYLATED HUMAN ALPHA INTERFERON MUTEINS, METHOD FOR OBTAINING THEM AND USE**

GLYKOSYLIERTE HUMANE ALPHA-INTERFERON-MUTEINE, VERFAHREN ZU IHRER GEWINNUNG UND ANWENDUNG

MUTÉINES DE L'INTERFERON ALPHA HUMAIN GLYCOSYLEES ET LEUR PROCÉDÉ D'OBTENTION ET D'UTILISATION

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **20.06.2006 AR P060102627**

(43) Date of publication of application:
**15.04.2009 Bulletin 2009/16**

(73) Proprietors:
• **Protech Pharma, S.A.**
**Santa Fe 3000 (AR)**
• **López, Ricardo Agustin**
**E-03002 Alicante (ES)**

(72) Inventors:
• **LÓPEZ, Ricardo Agustín**
**03002 Alicante (ES)**
• **CEAGLIO, Natalia Analia**
**Santo Tomé, Santa Fe (AR)**
• **ETCHEVERRIGARAY, Marina**
**3000 Santa Fe (AR)**
• **OGGERO EBERHARDT, Marcos Rafael**
**Rafaela, Santa Fe (AR)**
• **LOPEZ, Ricardo Agustin**
**03002 Alicante (ES)**
• **KRATJE, Ricardo**
**3000 Santa Fe (AR)**

(74) Representative: **Gislon, Gabriele**
**Torner, Juncosa i Associats, S.L.**
**C/Gran Via de les Corts Catalanes, 669bis 1è 2Ọ**
**08013 Barcelona (ES)**

(56) References cited:
**WO-A1-2004/045648**    **WO-A2-2004/019856**
**WO-A2-2006/020580**    **US-A1- 2005 266 465**

• **RAPP J C ET AL: "BIOLOGICALLY ACTIVE HUMAN INTERFERON ALPHA-2B PRODUCED IN THE EGG WHITE OF TRANSGENIC HENS", TRANSGENIC RESEARCH, LONDON, GB, vol. 12, no. 5, 1 October 2003 (2003-10-01), pages 569-575, XP009029972, ISSN: 0962-8819, DOI: DOI: 10.1023/A:1025854217349**
• **RADHAKRISHNAN R ET AL: "Zinc Mediated Dimer of Human Interferon-a2b Revealed by X-Ray Crystallography", STRUCTURE, CURRENT BIOLOGY LTD., PHILADELPHIA, PA, US, vol. 4, no. 12, 1 December 1996 (1996-12-01), pages 1453-1463, XP003013146, ISSN: 0969-2126, DOI: DOI:10.1016/S0969-2126(96)00152-9**
• **ADOLF G R ET AL: "NATURAL HUMAN INTERFERON-ALPHA2 IS O-GLYCOSYLATED", BIOCHEMICAL JOURNAL, THE BIOCHEMICAL SOCIETY, LONDON, GB, vol. 276, no. 2, 1 January 1991 (1991-01-01), pages 511-518, XP009020734, ISSN: 0264-6021**

**(Cont. next page)**

- **WILLIAMS R.W.: 'Secondary structure of human leukocyte interferon from Raman spectrometry' THE JOURNAL OF BIOLOGICAL CHEMISTRY vol. 260, no. 7, 10 April 1985, pages 3937 - 3940, XP008101903**

2

**Description**

FIELD OF THE INVENTION

The present invention belongs to the field of molecular biology related to the development of recombinant proteins. In particular, it is related to human interferon-alpha recombinant muteins having improved pharmacological, chemical and physical properties; process for obtaining said muteins and pharmaceutical formulations for use in mammals, particularly humans, in need of a therapeutic treatment with interferon. More particularly, this invention is related to human interferon-alpha muteins, containing at least one modified amino acid at a position pertaining to an alpha-helix-type secondary structure. Said modification generates at least one sequence of the Asn-Xaa-Ser/Thr type, introducing a N-glycosylation susceptible site by glycosylation of an asparagine residue in said sequence.

STATE OF THE ART

The group of cytokines known as interferons was characterized for the first time in 1957. They were designated as interferons (IFNs) because of their ability to interfere in viral replication, thereby conferring resistance to infection from infected cells to non-infected cells. Isaacs and Lindermann (Proc R Soc Lond Biol Sci, Sep 1957; 147(927): 258-67) were the first to demonstrate this characteristic in said cytokines.

IFNs are divided in two groups according to their structural and functional properties: type I interferons (IFN-alpha, IFN- beta, IFN- omega) and type II interferons (IFN- gamma) . Each of these is expressed in a great variety of cell types at low concentrations. IFN- alpha is expressed mainly in B- lymphocytes and macrophages; IFN- beta is expressed in fibroblasts, and IFN- gamma in T- lymphocytes.

Recently, a new member has been proposed for the interferon family, known as type III interferon, comprised by interferons lambda 1, 2, and 3 (corresponding to Interleukin- 29 (IL- 29), IL- 28a, and IL- 28b, respectively) (Kotenko, S. V. 2003. IFN- lambda mediate antiviral protection through .a distinct class II cytokine receptor complex. Nat. Immunol. 4: 69- 77; Sheppard, P. 2003. IL- 28, IL- 29 and their class II cytokine receptor IL- 28R. Nat. Immunol. 4: 63- 68) . These type III IFNs show a low degree of sequence homology to the $\alpha/\beta$ IFN family but they are activated by the same factors that induce expression of $\alpha/\beta$ IFNs and inhibit replication of several viruses including vesicular stomatitis virus (VSV) and encephalomyocarditis virus (EMVC) . In addition, they may also inhibit replication of type B and C hepatitis viruses.

Interferon- alpha belongs to a multigenic family consisting of genres and pseudogenes, of which more than 20 variants have been identified. The first three- dimensional models of the human type I IFNs was predicted in 1982 keeping in mind its amino acidic sequences. Recently, the tertiary structure of the IFN- alpha 2b was revealed by means of X-ray crystallography (Radhakrishnan et al. 1996. Zinc- mediated dimer of human interferon- alpha 2b revealed by X- ray crystallography. Structure 4 (12) : 1453- 1463), in order to fully describe the state of the art to which this invention pertains, while the corresponding to IFN- alpha 2a in solution was analyzed by means of NMR spectroscopy (Klaus et al. 1997. The three- dimentional high resolution structure of human interferon- alpha 2a determined by heteronuclear NMR spectroscopy in solution. J. Mol. Biol. 274: 661- 675), in order to fully describe the state of the art to which this invention pertains.

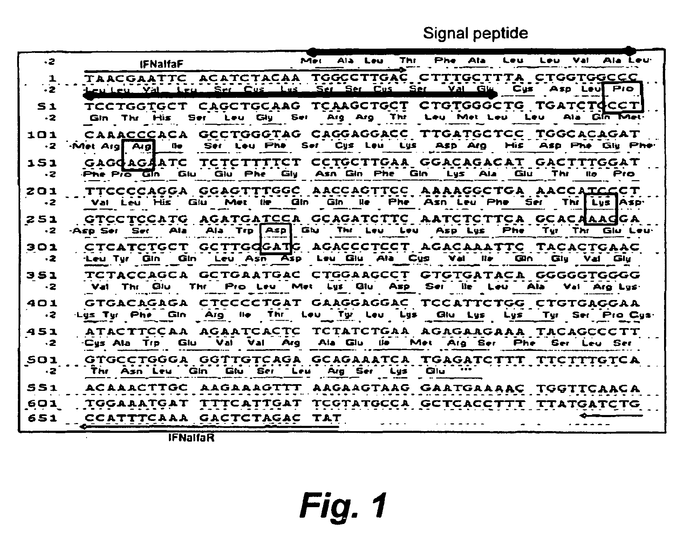

These papers demonstrated that the IFN- alpha2 possesses a consistent globular structure in 5 alpha helices designated with the letters A (Ser11- Met21), B and B' (Thr52- Ser68 and Lys70- Ala75), C (Glu78- Ile100), D (Leu110- Glu132) and E (Pro137- Leu157), which allows to classify this protein inside the group of the alpha- helical cytokines. The helices are linked by one long connection (AB loop) and 3 short segments (BC, CD and DE loops) . Each helix is approximately straight, except for helix B that contains a pronounced bend of 70° centered on Thr69, allowing to divide the mentioned helix in the region B and B' (Figure 1) .

The structure of interferon-alpha 2b contains 2 disulphide bonds and an O-linked glycosydic chain, at position Thr 106. The post-translational modifications necessary for transferring a glycan to protein take place in the cell's endoplasmic reticulum immediately after translocation of the freshly synthesized polypeptide from the cellular cytoplasm. For a N-type glycosylation to occur, the presence of sequences of the Asn-Xaa-Ser/Thr type in the amino acid chain is required, where an N-glycosydic bond is formed between the amino group of the asparagine residue and an OH group of the carbohydrate.

The high-level expression of type I interferon in living organisms is induced by the presence of a viral infection. It may also be induced by a great variety of other non-viral agents, such as bacteria, mycoplasma and different polymers, for example, membrane lipopolysaccharides of Gram-negative bacteria and the presence of synthetic polymers (Merigan T: Induction of circulating interferon by synthetic anionic polymers of known composition. Nature 1967, 214: 416-417).

Type I-interferon also exhibits anti-proliferative and immunomodulating activity.

The signaling pathway of type I IFNs starts with the binding of said molecule to the cellular surface receptor target and mediates the activation of target genes in the nucleus through the Jak/STAT signaling pathway. There are other gene activation pathways resulting from binding to the IFN-receptor that do not depend on the above-mentioned signal transduction pathway (A Lamer: Interferon signal transduction. Biotherapy, January 1, 1996; 8 (3-4): 175-81).

Interferon alpha is used clinically in the treatment of chronic hepatitis B and C, acute viral encephalitis, cancers such as nasopharyngeal carcinoma, lymphoma, leukemia, and melanomas, among others.

One of the major drawbacks of the use of interferon in the treatment of diseases is its low stability when administered to a living organism, and therefore it is necessary to provide excess amounts of interferon to achieve suitable therapeutic levels. In the first place, this contributes to the high cost of interferon therapies, particularly of Interferon-alpha therapies, and in the second place, in certain patients these treatments have side-effects such as flu-like conditions, fever, fatigue, irritability, chills, headaches and muscle pain; upset stomach, loss of appetite, diarrhea, dizziness, etc. (Richard Grieve: Cost-effectiveness of interferon or peg-interferon with ribavirin for histologically mild chronic hepatitis C. Gut, Jun 2005; 10.1136/gut.2005.064774; Gary L. Davis: Interferon-alpha-2b alone or with ribavirin for the treatment of relapse of chronic hepatitis C. 1998. Volume 339 Number 21-1493).

Currently new strategies are being developed with the aim to increase *in vivo* stability of these bioactive proteins using enzymatic reactions (WO 98/13381, US 6, 620, 916, US 5, 643, 564, US 4, 184, 917), chemical reactions (WO 96/21468, US 2004/0180054, US 6, 524, 570, US 4, 179, 337, US 5, 981, 709, US 20060029573, US 5, 738, 846), encapsulation of protein (WO2005074892), directed mutagenesis (US2004/0002474, WO92/01055, US 2005/0019871), etc. The purpose of these investigations is to achieve therapeutically effective doses not requiring the administration of large amounts of protein. Examples of these new strategies are disclosed in documents such as Patent WO98/13381 (Ajinomoto Kk (Jp) ; Takahara AND., et al.), that reveals a method for modifying biologically active proteins and enhancing its *in vivo* activity, by the addition of branched ligands to glutamine residues present in the primary sequence of the protein. Patent documents US 6, 620, 916 (Ajinomoto Co. Inc.; Takahara, et al.) ; US 5, 643, 564 (Takeda Chemical Industries, Ltd; Hamaguchi, et al.), and US 4, 184, 917 (Sandoz Ltd.; Dorner, et al.) describe methods to obtain interferon homologues with enhanced stability, by enzymatic modifications which alter their glycosylation profile. These new homologues act essentially in the liver and could improve effectiveness in this organ, but decrease the effect of interferon on other locations. Patent document WO 96/21468 (Amgen Inc; Collins David, et al.) reveals recombinant interferon- alpha analogues obtained by addition of glycosydic ligands with multiple lactoses conjugated to Arginine residues or to amino terminal residues of the natural interferon- alpha primary sequence. The product claimed in this document is directed to the liver and therefore would be acting at hepatic level and showing deficiencies in other organs such as, for example in kidney tumors. US Patent Application 2004/0180054 (Kim, Young- Min, et al. ) reveals a conjugate comprising a protein (IFN- alpha), a non- peptidic polymer, and an immunoglobulin, thus increasing the *in vivo* stability of the protein. In Patent documents US 4, 179, 337, US 5, 981, 709, US 20060029573, US 5, 738, 846, US 6, 524, 570; the inventors disclose processes, compositions, and uses of interferon- alpha conjugated to polyethyleneglycol for increasing product stability. These methods comprise purification steps that are necessary for removing reaction products before carrying out the minimum necessary purification steps for obtaining a high purity product. In addition, the proposed process shows the difficulty of directing a chemical reaction to a specific site of the protein, which leads to heterogeneous products, entailing the possibility of obtaining inactive products.

In Patent Application WO 2005074892 (Shenzhen Neptunus Interlong BI; Wang et al.) the inventors describe a cream containing interferon liposomes used for the treatment of skin diseases caused by viral infections (for example, herpes zoster), warts, genital ulcers, etc. This kind of preparation has the disadvantage of its limited application for the topical treatment of diseases.

US Patent Application 2004/0002474 (Maxygen, Inc.; Heinrichs, Volker, et al.), claims a method to obtain interferon-alpha homologues by modifications in the gene or amino acid sequence. In Patent document WO 92/01055 (Boehringer Ingelheim Int; Adolf Guenther, et al.) the inventors disclose a method for obtaining interferon-alpha homologues having new O-glycosylation sites. The product obtained by these strategies shows no improvements in pharmacokinetic properties as compared to pegylated interferon.

US Patent Application 2005/0019871 (Lee, Eun Jung, et al.) reveals a method for obtaining interferon-alpha isoforms, in which the nucleotide sequence has been modified in order to introduce Asn-Xaa-Ser/Thr motifs in sites that are not involved in forming secondary structures, and proposes sites located exclusively outside the alpha-helix structures. Although recombinant human interferon-$\alpha$2b isoforms with new N-glycosylation sites were obtained, there is no sign of improvement in stability and/or release over time profiles, as compared to known commercially available isoforms such as interferon pegylated with 12000 Dalton Polyethyleneglycol (PEG 12000). This application only describes isoforms with only two new N-glycosylation sites in molecular regions lacking alpha-helix structures, which would represent areas of greater susceptibility to the action of N-glycanase.

The present invention solves the problems described in the prior art and provides a preparation comprising a recombinant human interferon-alpha mutein, having an N-glycosylation profile which is different from those known in the prior art. Said mutein shows improved release over time and stability profiles, and an *in vivo* biological activity comparable to that of interferon pegylated with a 12000 Dalton Polyethyleneglycol (PEG 12000). In addition said mutein is obtained by a process comprising few steps and simple purification, yielding a high purity final product

OBJECTS OF THE INVENTION

It is a main object of the present invention to provide a recombinant human interferon- alpha mutein, preferably of the 2b type, containing at least one glycosylation site, preferably N- glycosylation, at a position of its amino acid sequence that forms part of an alpha- helix- type secondary structure, said recombinant human interferon- alpha mutein 2b further exhibits at least one N- glycosylation site at a position located outside an alpha- helix structure. Said N- glycosylation sites, comprising a consensus sequence of the Asn- Xaa- Ser/Thr- type, at a position of its amino acid sequence that is part of an alpha- helix- type secondary structure, are positions selected from the group consisting of: Leu9, Arg12, Asn65, Leu66, Phe67, Lys70, Asp71, Phe84, Asn93, Glu113, Arg125, Met148, Ser150, Ser152, Leu153, Asn156. While said N- glycosylation sites located outside alpha- helix- type secondary structures occupy positions selected from the group consisting of the following positions in natural human interferon- alpha 2b, Pro4, Thr6, Arg23, Leu26, Asn45, Ala50, Asp77, Gly104, Thr106, Lys134, Gln158, Leu161.

It is another object of the present invention to provide a gene encoding a recombinant human interferon-alpha mutein, preferably of type 2b, comprising at least one site-directed mutation that generates one N-glycosylation site, a consensus sequence of the Asn-Xaa-Ser/Thr type, involved in an alpha-helix structure. Said site-directed mutation is carried out in amino acids selected from the group consisting of Leu9, Arg12, Leu66, Phe67, Lys70, Asp71, Phe84, Leu95, Glu113, Arg125, Met148, Ser150, Ser152, Leu153, Asn156 amino acids said gene further exhibits at least one site-directed mutation that generates a consensus sequence of the Asn-Xaa-Ser/Thr type, where the Asn can be bound to an oligosaccharide through an N-glycosydic bond and is located outside an alpha-helix-type secondary structure. Said N-glycosylation site located outside an alpha-helix-type secondary structure is located in amino acids selected from the group consisting of Pro4, Thr6, Arg23, Leu26, Phe47, Ala50, Asp77, Gly104, Thr106, Lys134, Gln158, Leu161.

Claimed recombinant human interferon-alpha mutein may be obtainable by a method for generating new sites capable of being N-glycosylated using a site-directed mutagenesis procedure in the gene that encodes said recombinant human interferon-alpha mutein of the invention.

Claimed recombinant human interferon-alpha mutein may be obtainable by a method for obtaining an eukaryotic cell line, for producing said recombinant human interferon-alpha mutein of the invention by transformation or transfection of a cell line containing said gene encoding said recombinant human interferon-alpha mutein of the invention, inserted in a suitable expression vector the eukaryotic cell line is the CHO.K1 cell line.

Claimed recombinant human interferon-alpha mutein may be obtainable by a method for producing the recombinant human interferon mutein of the invention, said method comprising the steps of: a) culturing said transformed or transfected eukaryotic cell line with an expression vector containing the gene encoding the human interferon mutein of the invention, and b) isolating the expressed and secreted recombinant human interferon-alpha mutein from the culture medium.

Claimed recombinant human interferon-alpha mutein may be obtainable by a process for purifying said recombinant human interferon-alpha mutein of the invention by immunoaffinity chromatography.

Claimed recombinant human interferon-alpha mutein may be obtainable by a method for producing the human interferon mutein of the invention from prokaryotic cells comprising the steps of: a) transforming or transfecting a prokaryotic cell with a suitable expression vector containing the gene encoding the recombinant human interferon-alpha mutein of the invention; b) selecting a clone expressing the polypeptide of the recombinant human interferon-alpha mutein of the invention; c) culturing said clone, d) purifying e) glycosylating *"in vitro"* a polypeptide of the human interferon-alpha mutein expressed by the clone of step c); and f) purifying the human interferon-alpha mutein of the invention.

It is another object of the present invention to provide a pharmaceutical composition comprising at least the recombinant human interferon-alpha mutein of the invention for the treatment of diseases such as melanomas, chronic hepatitis C, acute and chronic hepatitis B, acute and chronic non-A, non-B hepatitis, Kaposi's sarcoma, multiple sclerosis, genital warts, leukemia, viral infections, among others.

It is another object of the present invention to provide a method for the treatment of melanomas, chronic hepatitis C, acute and chronic hepatitis B, acute and chronic non-A, non-B hepatitis, Kaposi's sarcoma, multiple sclerosis, genital warts, leukemia, viral infections, comprising the administration to a mammal; preferably a human, in need thereof, of an effective amount of a pharmaceutical composition comprising at least the recombinant human interferon-alpha mutein of the invention and wherein said composition further comprises pharmacologically acceptable carriers.

It is another object of the present invention to provide the use of the recombinant human interferon-alpha mutein of the invention for manufacturing a medicament for the treatment of chronic hepatitis C, using a therapeutic protocol in which the usual therapeutic doses of interferon are reduced.

BRIEF DESCRIPTION OF THE INVENTION

The present invention provides a recombinant human interferon-alpha 2b mutein, comprising at least one consensus sequence of the Asn-Xaa-Ser/Thr N-glycosylation site where said site is generated by the substitution of an amino acid located in an alpha helix-type structure, and said site is Lys70; the group consisting of positions Leu9, Arg12, Asn65, Leu66, Phe67, Phe84, Asn93, Glu113, Arg125, Met148, Ser150, Ser152, Leu153, Asn156 are not within the scope of the invention said sites are selected from the group consisting of Lys70, Asn93, Glu113.

In a preferred embodiment, said recombinant human interferon-alpha 2b mutein of the present invention contains a N-glycosylation site and stability to N-glycanases of at least 70% regarding the initial concentration after 300 minutes of reaction.

said recombinant human interferon-alpha 2b mutein further has at least one glycosylation site in a position located outside an alpha helix-type structure and said sites are selected from the group consisting of positions Pro4, Thr6, Arg23, Leu26, Asn45, Ala50, Asp77, Gly104, Thr106, Lys134, Gln158, Leu161.

In another preferred embodiment, said recombinant human interferon-alpha 2b mutein comprises two N-glycosylation sites; preferably, it has one N-glycosylation site located at position Lys70 and one N-glycosylation site located outside an alpha-helix-type secondary structure, selected from the group consisting of positions Pro4, Arg23 and Asp77.

In another embodiment, said recombinant human interferon-alpha. 2b mutein comprises three N-glycosylation sites. Preferably, it has one N-glycosylation site located at position Lys70 and two N-glycosylation sites selected from the group consisting of positions Pro4, Arg23 and Asp77.

In another preferred embodiment, said recombinant human interferon-alpha 2b mutein comprises four N-glycosylation sites and a plasmatic clearance, of 0.36 ml/min or less. Preferably, said mutein comprises glycosylation sites Pro4, Arg23, Lys70 and Asp77.

Recombinant human interferon-alpha 2b mutein comprises five N-glycosylation sites. Preferably, said sites are selected from the group consisting of Lys70, Pro4, Arg23, Asp77 Asn93 and Glu113 (not within the scope of the present invention).

In another preferred embodiment, the present invention provides a gene encoding recombinant human interferon-alpha 2b mutein comprising at least one consensus sequence of the Asn-Xaa-Ser/Thr N-glycosylation site where said site is generated by a site-directed mutation that generates said N-glycosylation site in an alpha-helix-type structure, and said site is Lys70; the group consisting of positions Leu9, Arg12, Asn65, Leu66, Phe67, Phe84, Asn93, Glu113, Arg125, Met148, Ser150, Ser152, Leu153, Asn156 are not within the scope of the present invention.

In another preferred embodiment, said gene presents site-directed mutations so as to generate the substitution of an amino acid and said gene encodes recombinant human interferon-alpha 2b mutein with one N-glycosylation site, and said mutain is stable in the presence of N-glycanase at least 70% of its initial concentration, after 300 minutes of reaction. The gene according to the present invention presents site-directed mutations so as to generate the substitution of the amino acid at position Lys70.

In another preferred embodiment, the gene of the present invention further exhibits at least one site-directed mutation that generates a consensus sequence of the Asn-Xaa-Ser/Thr type, where the Asn may be bound to an oligosaccharide through an N-glycosydic bond and is located outside an alpha-helix-type secondary structure said site-directed mutation, that generates a N-glycosylation site located outside an alpha-helix-type secondary structure takes place in amino acids selected from the group consisting of Pro4, Asp77, Azg23; Thr6, Leu26, Phe47, Ala50, Leu66, Phe67, Gly104, Thr106, Lys134, Gln158, Leu161 are not within the scope of the present invention.

In another preferred embodiment, the gene of the present invention comprises site-directed mutations that modify two amino acid positions. Preferably, said mutations take place in the amino acid corresponding to Lys70 position and in an amino acid selected from the group consisting of amino acids Pro4, Arg23 and Asp77.

In another preferred embodiment, the gene of the present invention comprises site-directed mutations that modify three amino acid positions. Preferably, said site-directed mutations take place in the amino acid corresponding to Lys70 position and in two of the amino acids selected from the group consisting of amino acids Pro4, Arg23 and Asp77.

In another preferred embodiment, the gene of the present invention comprises site-directed mutations that modify four amino acid positions. More preferably, said site-directed mutations take place in the amino acid corresponding to Lys70, Pro4, Arg23 and Asp77 positions.

The gene comprises site-directed mutations that modify five amino acid positions. Said mutations take place at the amino acids selected from the group consisting of amino acids Lys70, Pro4, Arg23, Asp77, Leu95 and Glu113 (not within the scope of the present invention).

A method for producing the gene encoding human interferon mutein of the invention where said method comprises the steps of: a)- cloning a gene encoding human interferon-alpha 2b in an appropriate vector for its amplification, b)- producing the gene encoding human interferon-alpha 2b mutein of the present invention using the site-directed mutagenesis technique, c)- cloning the genetically modified gene from step b, into a suitable eukaryotic expression vector.

A method for obtaining a eukaryotic cell line which produces the recombinant human interferon-alpha 2b mutein of the present invention wherein said method comprises the steps of: a)- transforming or transfecting said eukaryotic cell with said eukaryotic expression vector, b)- selecting that clone expressing the recombinant humain interferon-alpha 2b mutein of the present invention. the eukaryotic cell line comprises a cell line derived from CHO.K1 line.

A process for producing the recombinant human interferon-alpha 2b mutein of the present invention comprising the steps of: a)- culturing said eukariotyc cell transformed or transfected with an expression vector containing the gene encoding recombinant human interferon-alpha mutein of the present invention, b)- isolating the recombinant human interferon-alpha 2b mutein secreted into the culture medium.

It is also disclosed a procedure for purifying the human interferon mutein of the present invention starting from the culture medium mentioned above, wherein said mutein purification is done by immunoaffinity chromatography.

A method for producing the gene, according to the present invention, encoding human interferon mutein of the invention and said method comprises the steps of: a)- cloning a gene encoding human interferon-alpha 2b in an appropriate vector for its amplification, b)- producing the gene encoding human interferon-alpha 2b mutein of the present invention using the site-directed mutagenesis technique, c)- cloning the genetically modified gene from step b, into a suitable prokaryotic expression vector.

A process for obtaining the recombinant human interferon-alpha 2b mutein of the present invention is also disclosed wherein said process comprises the steps of: a)-transforming or transfecting a prokaryotic cell with suitable prokaryotic expression vector, b) - selecting that clone expressing the polypeptide of the recombinant human interferon-alpha 2b mutein of the present invention, c)-culturing said clone transformed or transfected with said suitable prokaryotic expression vector containing the gene encoding recombinant human interferon-alpha 2b mutein of the present invention, d) purifying the polypeptide of the human interferon-alpha 2b mutein of the present invention, e) - glycosylating in vitro the polypeptide of the human interferon-alpha 2b mutein of the present invention, f) purifying the recombinant human interferon-alpha 2b mutein of the present invention.

In another preferred embodiment of the present invention, a pharmaceutical composition is provided, comprising at least one recombinant human interferon-alpha 2b mutein of the present invention. In another preferred embodiment, said formulation further comprises pharmacologically acceptable excipients. Said pharmaceutical composition is administered for the treatment of melanoma, chronic hepatitis C, acute and chronic hepatitis B, acute and chronic non-A, non-B hepatitis, Kaposi's sarcoma, multiple sclerosis, genital warts, leukemia, viral infections. Preferably, said composition is administered to a mammal; preferably a human, at a dose between 0.1 and 2.0μg/kg weekly. Preferably, the composition is administered in combination with ribavirin.

In another preferred embodiment, the present invention provides a method for the treatment of melanoma, chronic hepatitis C, acute and chronic hepatitis B, acute and chronic non-A non-B hepatitis, Kaposi's sarcoma, multiple sclerosis, genital warts, leukemia, viral infections, said method comprises administration to a mammal, preferably human, in need thereof, an effective quantity of a pharmaceutical composition comprising at least one recombinant human interferon-alpha 2b mutein of the present invention, wherein said formulation further comprises pharmacologically acceptable excipients. Preferably, said method of treatment comprises the administration of the recombinant human interferon-alpha 2b mutein of the present invention by a route of administration selected from the group consisting of subcutaneous, parenteral, oral, sublingual, intranasal, topic routes. More preferably, the treatment method comprises the administration of the recombinant human interferon-alpha 2b mutein of the present invention at a dose between 0.1 and 2.0 μg/kg weekly.

In another preferred embodiment, the present invention provides using the recombinant human interferon-alpha 2b mutein of the present invention for manufacturing a medicament for the treatment of melanoma, chronic hepatitis C, acute and chronic hepatitis B, acute and chronic non-A, non-B hepatitis, Kaposi's sarcoma, multiple sclerosis, genital warts, leukemia, viral infections. Said medicament is administered at a dose between 0.1 and 2.0μg/kg weekly, more preferably, at a dose between 0.5 and 1.0 μg/kg weekly in combination with ribavirin, and it is administered by a route of administration selected from the group consisting of subcutaneous, parenteral, oral, sublingual, intranasal, topic routes.

BRIEF DESCRIPTION OF THE FIGURES

Figure 1a: shows the amino acid sequence of human type I interferon-alpha 2b, human interferon-alpha 1, human interferon-beta and murine interferon-beta. The highlighted amino acids in the consensus sequence correspond to the sequences conserved in the human alpha interferons, while the residues highlighted in the rest of the sequences are conserved in all type I interferons. The alpha helix-type secondary structure corresponding to the molecules of human interferon alpha 2b and murine interferon beta are indicated as segments in the upper and lower part of the respective sequences (adapted from Radhakrishnan et al. 1996. Zinc-mediated dimer of human interferon - alpha2b revealed by X-ray crystallography. Structure 4 (12): 1453-1463).

Figure 1b: nucleotidic sequence of the interferon alpha 2b. The thin lines indicate the codons corresponding to the first amino acid of the protein sequence (Cys), the last amino acid of this sequence (Glu) and the STOP codon.

Figure 2: Site-directed mutagenesis using an overlapping extension PCR technique.

Figure 3: Western blot Analysis. Lanes: 1- Molecular mass marker; 2- rhIFN-α2bM4; 3- rhIFN-α2bM77; 4- rhIFN-α2bM23; 5-rhIFN-α2bM70; 6- O-glycosylated rhIFN-α2b; 7- non-glycosylated rhIFN-α2b; 8- rhIFN-α2bM4/23/70/77.

Figure 4: Enzymatic N- deglycosylation of mutated variants of hIFN- a2b (hIFN- α2bM23, hIFN- α2bM47, hIFN-α2bM70, hIFN- α2bM95) .

Figure 5: Percent variation in time of N-glycosylation expressed as N-glycosylation percent of the amount present at the beginning of the enzymatic deglycosylation procedure.

Figure 6 (A and B): SDS-PAGE under reducing conditions with silver staining (A) and Western blot (B) corresponding to purification of rhIFN-α2bM77 and rhIFN- α2bM4/23/70/77 by immunoaffinity chromatography. Lanes: 1- Raw sample of a culture supernatant containing rhIFN-α2bM77 before purification; 2-Purified sample of rhIFN-α2bM77; 3- Raw sample of a culture supernatant containing rhIFN-α2bM4/23/70/77 before purification; 4- Purified sample of rhIFN-α2bM4/23/70/77.

Figure 7: Time profile of IFN biological activity variations in each sample after subcutaneous inoculation of wild type rhIFN α2b, rhIFN α2bM23, and rhIFN α 2bM70 variants.

Figure 8: Time profile of percent residual biological activity of IFN intravenously administered to each rat for non-glycosylated rhIFN-α2b, rhIFN-α2bM77, rhIFN-α2b-PEG (12kDa), and rhIFN-α2bM4/23/70/77 variants.

Figure 9: Time profile of biological activity for IFN present in each sample after subcutaneous inoculation with non-glycosylated rhIFN-α2b, rhIFN-α2b-PEG (12kDa), and rhIFN-α2bM4/23/70/77 variants.

## DETAILED DESCRIPTION

Some terms used in the present specification are defined below to facilitate understanding of the invention:

"Natural human interferon- alpha 2b" (hIFN- α2b), refers to a cytokine as it is found in nature, without having been subjected to any kind of artificial modification.

"Amino acid substitution", refers to the change of one amino acid in the primary sequence of hIFN-α2b for another amino acid.

"Recombinant human interferon-alpha mutein of the invention", refers to recombinant molecules of human interferon-alpha, preferably alpha 2b, containing at least one glycosylation site, preferably N-glycosylation, at a position of its amino acid sequence forming part of an alpha-helix-type secondary structure.

"Human interferon-alpha" includes analogues, mutants, isoforms, and fragments of natural interferon-alpha.

"N- glycosylation site", refers to an Asn- Xaa- Ser/Thr tripeptide, where X may be any residue except a proline residue. The "position" of the "N- glycosylation site" is indicated by the position occupied by an amino acid residue in the amino acid sequence of a natural human interferon- alpha 2b that will be replaced by an Asn or it is the asparagine of said consensus sequence. Said Asn residue, in said consensus sequence, may be subjected to an N- type enzymatic glycosylation.

Glycosylation of certain eukaryotic proteins takes place at certain positions of the polypeptide backbone, and commonly there are two types of glycosylation. O- type glycosylation involves binding of an oligosaccharide to an- OH group of a serine or threonine residue, and N- type glycosylation, which involves binding of an oligosaccharide to an- NH group of an Asparagine residue. Particularly, N- glycosylation takes place in the consensus sequence, Asn- X- Ser/Thr, where X may be any amino acid different from Proline. All the oligosaccharides bound to a protein through an N- type binding have a pentasaccharide nucleus in common comprised by three mannose residues and two N- acetylglucosamine residues. Any sugars bound to this pentasaccharide nucleus may acquire a great variety of oligosaccharide patterns. The presence or absence of said oligosaccharides affects the physical properties of proteins and may be critical in their function, stability, secretion, and location in the cell.

the production of a human interferon- alpha mutein having at least one amino acid substitution in the sequence of the human natural interferon- alpha at a position forming part of an alpha- helix secondary structure, resulting in the consensus sequence Asn- Xaa- Ser/Thr, where the Asn residue is capable of being N- glycosylated. the recombinant human interferon- alpha mutein of the invention, containing at least  one N- glycosylation site at a position forming part of an alpha- helix secondary structure, is obtained by an amino acid substitution at position Lys70 with an Asn residue (Lys70Asn) . This generates the consensus sequence, Asn- Xaa- Ser/Thr, where the asparagine residue is part of an alpha- helix secondary structure and is susceptible to N- glycosylation. Another example may be the substitution of the amino acid residue Leu95 with a Serine or Threonine residue, thus generating a consensus sequence, Asn- Xaa- Ser/Thr, wherein the asparagine residue at position Asn93 is susceptible to N- glycosylation (not within the scope of the present invention) . It has been demonstrated, that the presence of at least one N- glycosylation site in alpha- helix- type structures confers a greater stability to interferon and therefore a longer half- life in blood.

Said N-glycosylation sites, generated by substitution of an amino acid forming part of an alpha-helix-type secondary structure are selected from the group consisting of amino acid positions Leu9, Arg12, Asn65, Leu66, Phe67. Asp71, Phe84, Asn93, Glu113, Arg125, Met148, Ser150, Ser152, Leu153, Asn156 (not within the scope of the present invention).

One preferred embodiment of the present invention is a recombinant human interferon-alpha mutein having at least one amino acid substitution at a position forming part of an alpha-helix secondary structure and in addition at least one substitution in an amino acid at a position located outside the alpha-helix structure so that Asn-Xaa-Ser/Thr consensus sequences are obtained, where the Asn residue is susceptible to N-glycosylation. Said N-glycosylation sites at positions located outside alpha-helix structures are selected from the group consisting of amino acid positions Pro4, Arg23, Asp77; Thr6, Leu26, Asn45, Ala50, Gly104, Thr106, Lys134, Gln158, Leu161 (not within the scope of the present invention).

In a preferred embodiment, the recombinant human interferon-alpha mutein of the invention contains in its amino acid sequence an N-glycosylation site at a position forming part of alpha-helix structures, wherein the position of the glycosylation site is Lys70; the group consisting of amino acid positions Leu9, Arg12, Asn65, Leu66, Phe67, Asp71, Phe84, Asn93, Glu113, Arg125, Met148, Ser150, Ser152, Leu153, Asn156 (not within the scope of the present invention). According to the invention, the position is Lys70; Asn93, Glu113 (not within the scope of the present invention). According to the invention, the position is Lys70.

According to the invention, said mutein contains a glycosylation site at position Lys70 and further an N-glycosylation site at a position selected from the group consisting of positions Pro4, Arg23, and Asp77. Additional N-glycosylation sites (not according to the invention) are selected from the group consisting of amino acid positions Leu9, Arg12, Asn65, Leu66, Phe67, Asp71, Phe84, Asn93, Glu113, Arg125, Met148, Ser150, Ser152, Leu153, Asn156, Thr6, Leu26, Asn45, Ala50, Gly104, Thr106, Lys134, Gln158, Leu161.

The recombinant human interferon-alpha mutein contains three N-glycosylation sites selected from the group consisting of amino acid positions Leu9, Arg12, Asn65, Leu66, Phe67, Asp71, Phe84, Asn93, Glu113, Arg125, Met148, Ser150, Ser152, Leu153, Asn156, Thr6, Asn45, Leu26, Ala50, Gly104, Thr106, Lys134, Gln158, Leu161 (not according to the invention) the recombinant human interferon-alpha mutein of the invention having three N-glycosylation sites, contains one N-glycosylation site at position Lys70 and two N-glycosylation sites selected from the group consisting of positions Pro4, Arg23, and Asp77.

The recombinant human interferon-alpha mutein of the invention is a mutein containing four N-glycosylation sites said sites are Pro4, Arg23, Lys70, and Asp77 the group consisting of amino acid positions Leu9, Arg12, Asn65, Leu66, Phe67, Lys70, Asp71, Phe84, Asn93, Glu113, Arg125, Met148, Ser150, Ser152, Leu153, Asn156, Pro4, Thr6, Arg23, Leu26, Asn45, Ala50, Asp77, Gly104, Thr106, Lys134, Gln158, Leu161 (not within the scope of the invention).

The recombinant human interferon-alpha mutein contains 5 N-glycosylation sites selected from the group of amino acid positions, Leu9, Arg12, Asn65, Leu66, Phe67, Lys70, Asp71, Phe84, Asn93, Glu113, Arg125, Met148, Ser150, Ser152, Leu153, Asn156, Pro4, Thr6, Arg23, Leu26, Asn45, Ala50, Asp77, Gly104, Thr106, Lys134, Gln158, Leu161. said sites are selected from the group consisting of positions Pro4, Arg23, Lys70, Asp77, Asn93, Glu113.

In order to obtain the recombinant human interferon-alpha mutein of the invention containing sites susceptible to N-glycosylation, two different strategies (not within the scope of the present invention) were followed:

1) locating all serines and/or threonines of the hIFN-α2b amino acid sequence and changing the amino acid occupying the first position of the consensus sequence by an asparagine,

2) as an alternative strategy, locating all asparagines present in the hIFN-a2b sequence and substituting the amino acid at the third position by a serine or threonine.

The modifications carried out in the natural amino acid sequence of human interferon-alpha for obtaining the recombinant mutein of the invention are a result of a genetic modification of the gene encoding natural human interferon-alpha 2b. Further, said genetic modifications are introduced in such a way that they generate an Asn-Xaa-Ser/Thr consensus sequence in the amino acid sequence of the human interferon-alpha, wherein the Asn residue is susceptible to N-glycosylation.

The gene encoding the recombinant human interferon-alpha mutein of the invention, claimed hereunder, comprises at least one genetic modification in a codon of the gene encoding human natural interferon-alpha. Said modification generates the Asn-Xaa-Ser/Thr consensus sequence at a position that is a part of an alpha-helix secondary structure, thereby generating an N-glycosylation site at a position forming part of an alpha-helix-type secondary structure. According to the invention said modification of the nucleotide sequence is carried out in at Lys70; the positions selected from the group consisting of amino acid positions Leu9, Arg12, Leu66, Phe67, Asp71, Phe84, Leu95, Glu113, Arg125, Met148, Ser150, Ser152, Leu153, Gln158 are not within the scope of the present invention.

According to the present invention, said gene encoding the recombinant human interferon-alpha mutein of the invention, further shows at least one genetic modification in a codon of the gene encoding the human natural interferon-alpha, such that said modification generates the Asn-Xaa-Ser/Thr consensus sequence at a position located outside an alpha-helix secondary structure. This generates an N-glycosylation site at a position located outside an alpha-helix-type secondary structure. Said modifications in positions  located outside alpha-helix-type secondary structures are carried out at positions selected from the group consisting of position Pro4, Arg23, Asp77, additional positions, Thr6, Leu26, Phe47, Ala50, Gly104, Thr106, Lys134, Gln158, Leu161 are not within the scope of the invention.

According to the invention said gene has only one modification in its nucleotide sequence which takes place in the codon encoding amino acids Lys70; the group consisting of Leu9, Arg12, Leu66, Phe67, Asp71, Phe84, Leu95, Glu113, Arg125, Met148, Ser150, Ser152, Leu153, Gln158 are not within the scope of the invention, it takes place in the codon encoding the amino acid at position Lys70.

According to the invention, the gene encoding the recombinant human interferon-alpha mutein of the present invention has two modifications in its nucleotide sequence such that two N-glycosylation sites are generated. modifications involve codons encoding amino acids selected from the group consisting of Leu9, Arg12, Leu66, Phe67, Asp71, Phe84, Leu95, Glu113, Arg125, Met148, Ser150, Ser152, Leu153, Gln158, Thr6, Leu26, Phe47, Ala50, Gly104, Thr106, Lys134, Leu161. According to the invention the modifications in its nucleotide sequence are in the codon encoding Lys70 and in the codon encoding one of the amino acids selected from the group consisting of Pro4, Arg23, and Asp77.

In another preferred embodiment of this invention, the gene encoding the recombinant mutein of the present invention has three modifications in its nucleotide sequence such that three N-glycosylation sites are generated. modifications involve codons encoding amino acids selected from the group consisting of Leu9, Arg12, Leu66, Phe67, Asp71, Phe84, Leu95, Glu113, Arg125, Met148, Ser150, Ser152, Leu153, Gln158, Thr6, Leu26, Phe47, Ala50, Gly104, Thr106, Lys134, Leu161 are not within the scope of the invention. According to the invention modifications in its nucleotide sequence are in the codon encoding Lys70 and in two codons encoding amino acids selected from the group consisting of Pro4, Arg23, and Asp77.

In a further more preferred embodiment of this invention, the gene encoding the recombinant mutein of the present invention has four modifications in its nucleotide sequence such that four N-glycosylation sites are generated. modifications involve the codons encoding amino acids selected from the group consisting of Leu9, Arg12, Leu66, Phe67, Asp71, Phe84, Leu95, Glu113, Arg125, Met148, Ser150, Ser152, Leu153, Gln158, Thr6, Leu26, Phe47, Ala50, Gly104, Thr106, Lys134, Leu161 are not within the scope of the invention. According to the invention, the modifications in its nucleotide sequence involve codons encoding Pro4, Arg23, Lys70, and Asp77.

The gene encoding the recombinant human interferon-alpha mutein of the invention has five modifications in its nucleotide sequence in codons encoding amino acids selected from the group consisting of Leu9, Arg12, Leu66, Phe67, Asp71, Phe84, Leu95, Glu113, Arg125, Met148, Ser150, Ser152, Leu153, Gln158, Pro4, Thr6, Arg23, Leu26, Phe47, Ala50, Asp77, Gly104, Thr106, Lys134, Leu161 are not within the scope of the invention. in amino acids selected from the group consisting of Pro4, Arg23, Lys70, Asp77, Leu95, and Glu113 are not within the scope of the invention.

a method for generating said sites susceptible to N-glycosylation comprises the generation of point mutations in the nucleotide sequence of the gene encoding the human natural interferon-alpha, by means of a site-directed mutagenesis technique in said gene. The method comprises the following steps:

a) cloning a gene encoding natural human interferon-alpha 2b in a suitable plasmid.
b) generating mutations required for producing the human interferon-alpha mutein of the invention using a site-directed mutagenesis technique, and
c) cloning the genetically modified gene from step b, into a suitable expression vector.

The expression vector is selected from the group of vectors capable of carrying the gene of the invention and further containing the necessary elements for expressing the gene of interest in eukaryotic cells. Said vector may be expression vector pCl-neo.

The site-directed mutagenesis technique of the invention involves the use of oligonucleotides specifically designed to that end. This technique comprises two stages. In the first stage, two PCR reactions are carried out separately using oligonucleotides that hybridize to the terminal ends of the fragment cloned into a pCl-neo vector (oligonucleotides designated IFNalphaF and IFNalphaR, Table I), and oligonucleotides carrying a point mutation of Table II *(mut a and mut b)* which hybridize to the internal region of the gene where the mutation is to be introduced. A reaction mixture is obtained in tube a using a reverse external oligonucleotide (IFNalphaR) and the direct oligonucleotide *mut a*. Another reaction mixture is obtained in tube *b* with a direct external oligonucleotides and the reverse oligonucleotide *mut b*. PCR products from both reactions are purified by agarose gel electrophoresis and used as a template for the second stage. This second stage comprises a second PCR reaction using direct and reverse external oligonucleotides. The first three cycles are carried out without the addition of primers to allow hybridization and elongation of the complete product *(fill in)* and finally these are added for the amplification.

To obtain more than one N-glycosylation site within the recombinant human interferon-alpha mutein of the invention, said muteins are constructed sequentially as follows: first, a mutein with one N-glycosylation site is generated, using a site-directed mutagenesis technique, and then said mutein is used as a starting template for generating a new N-glycosylation site.

The method for obtaining a derivate eukaryotic cell line which produces the recombinant human interferon-alpha mutein of the invention, a cell line CHO.K1, transfected or transformed with an expression plasmid pCl-neo containing the gene encoding the recombinant human interferon-alpha mutein of the invention, involves the following steps:

- transforming or transfecting an eukaryotic cell with an expression vector containing an insert of said gene encoding the recombinant human interferon-alpha mutein of the invention, and
- selecting said clone expressing the recombinant human interferon-alpha mutein of the invention.

A process, for obtaining the recombinant human interferon-alpha mutein of the invention from said CHO.K1 cell line, comprises the steps of:

a) culturing the recombinant human interferon-alpha mutein-producing eukaryotic cell of the invention, and
b) isolating the recombinant human interferon-alpha mutein of the invention expressed and secreted into the culture medium.

A method for purifying the recombinant human interferon-alpha mutein of the invention, involves obtaining specific monoclonal antibodies for said mutein, adsorbing said monoclonal antibodies on a suitable chromatographic column and purifying the mutein of the invention by immunoaffinity chromatography.

Interferon- alpha may be expressed in bacteria, yeasts or insect cells according to procedures well known in the art. In all these cases, the recombinant interferon either is not glycosylated or it has a degree of glycosylation that is lower and different from the glycosylated interferon produced in animal cells. In all these cases, sugar chains may be glycosylated or remodelled "*in vitro*". For this purpose, there exist numerous protocols described in detail in the following Patents or Patent Applications: WO03031464, WO9425615, WO9216640, US20030040037, US20030003529, US20020137134, US20020019342, US20030124645, US20020160460, US20020142370, US20020119516.

a method for producing the human interferon mutein of the comprising the steps of:

a) transforming or transfecting a prokaryotic cell with a suitable prokaryotic expression vector containing the gene encoding the recombinant human interferon-alpha mutein of the invention; b) selecting a clone expressing the polypeptide of the recombinant human interferon-alpha mutein of the invention; c) culturing said clone in a suitable culture medium, d) purifying the product, e) glycosylating "*in vitro*" the human interferon-alpha mutein polypeptide expressed by the clone of step c); and f) purifying the human interferon-alpha mutein of the invention.

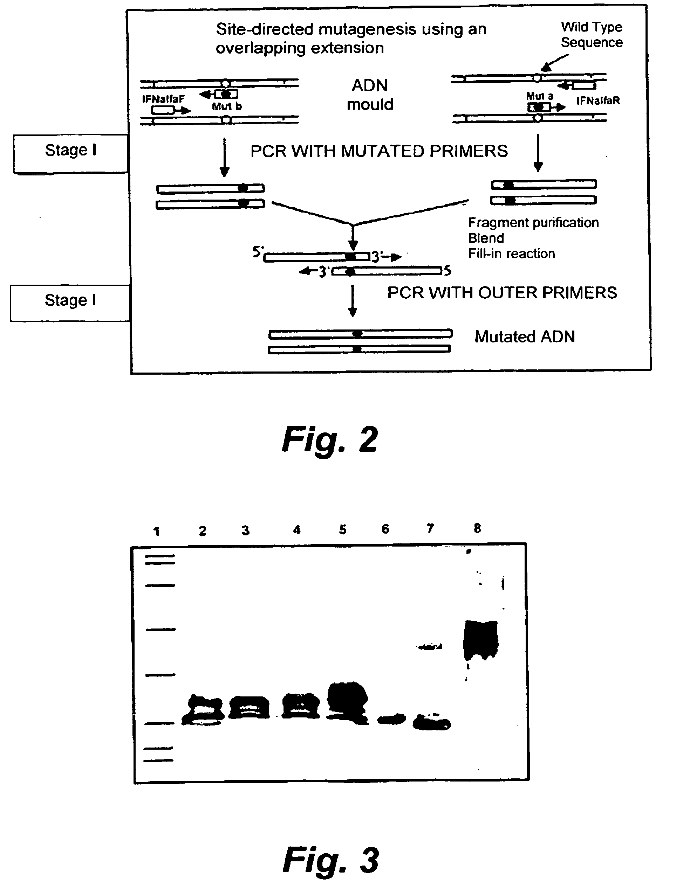

Glycosylation profiles of various recombinant hIFN-$\alpha$2b muteins were analyzed: muteins with one glycosylation site located outside an alpha-helix secondary structure; the recombinant human interferon-alpha mutein of the invention; natural hIFN-$\alpha$2b; and a non-glycosylated isoform; as shown in Figure 3. All mutants with only one site capable of being N-glycosylated showed a mixture of IFN-$\alpha$ isoforms of different molecular mass corresponding to the following fractions: non-glycosylated; O-glycosylated; and N-O-glycosylated; with a variable ratio of each fraction depending on the mutant being analyzed. In the particular case of the mutein whose gene contains a point mutation in the codon encoding the amino acid at position Lys70 such that it generates an N-glycosylation site in alpha-helix structure, rhIFN-$\alpha$2bM70, a greater molecular mass was observed as compared to muteins in which only one site capable of being N-glycosylated located outside alpha-helix-type structures was incorporated. Said mutein, rhIFN-$\alpha$2bM70, corresponds to a molecule exhibiting a larger content of glycosidic structures. Glycosylation in an alpha-helix structure confers greater resistance to the oligosaccharide linked to the Asn70 and as a consequence an increased stability against the action of N-glycanases. The same results are obtained when evaluating the stability of a mutein having one glycosylation site at position Asn93, which also belongs to an alpha-helix-type secondary structure (see example 7).

The recombinant human interferon-alpha mutein of the invention in a preferred embodiment comprising four N-glycosylation sites at positions Pro4, Arg23, Lys70, and Asp77, rhIFN-$\alpha$2bM4/M23/M70/M77, exhibits a homogeneous glycosylation profile with a high glycosylation value, as can be seen in figure 3. Further, in the same figure it can be seen that this mutein, rhIFN-$\alpha$2bM4/M23/M70/M77, has a low content of the O-glycosylated isoform and of those corresponding to a low glycosylation value. This means that a higher concentration of muteins is observed with a high occupation level within a range of molecular masses from 21 to 45 kDa, more particularly from 30 to 45 kDa.

By creating these new N- glycosylation sites it has become possible to increase the *in vivo* stability of cytokine as compared to any previously known isoform of rhIFN- $\alpha$2b. In a preferred aspect of the invention, the creation of the recombinant human interferon- alpha mutein of the invention with four N- glycosylation sites (rhIFN-$\alpha$2bM4/M23/M70/M77) displays a release over time profile equivalent to the commercially available molecule of interferon-PEG 12000, resulting in a product with a high degree of N- glycosylation and a more homogeneous glycosylation profile. In addition, notably the product thus obtained does not involve chemical reactions or the addition of synthetic polymers.

The resulting biological activity versus time values show that the recombinant human interferon- alpha mutein of the invention, in particular that corresponding to a mutein with a new N- glycosylation site at position Lys70, shows a 1, 63- fold increase of as compared to a mutein with a new N- glycosylation site lacking an alpha- helix structure (rhIFN-$\alpha$2bM23). This clearly indicates the importance of selecting N- glycosylation sites within structures of the alpha- helix type.

The recombinant human interferon-alpha mutein of the invention exhibits improved pharmacokinetic, physical, and chemical properties with respect to previously known isoforms obtained my means of cell cultures.

A further object of the present invention is a pharmaceutical formulation containing the recombinant human interferon- alpha mutein of the invention, for administration and treatment of diseases such as melanomas, chronic hepatitis C, acute and chronic hepatitis B, acute and chronic non- A, non- B hepatitis, Kaposi's sarcoma, multiple sclerosis, genital warts, leukemia, viral infections, among others. It also comprises the preparation of a powder, gel, cream, lyophilizate, tablets, or a solution to be administered by a route selected from the group consisting of subcutaneous, parenteral, oral, sublingual, intranasal, or topic administration. The formulation, in a preferred solution preparation, comprises concentrations from 0.02mg/ml to 3mg/ml of protein mass in solution. Said solution formulations which contain the interferon of the present invention, also contain a buffer, a stabilizer, a cryoprotectant, and a solvent. Useful buffers are those maintaining pH values from 4.5 to 7.5, preferably from 6.5 to 7.0, more preferably pH 6.8. The buffers that may be used are selected from citrate/ citric acid, acetate/ acetic acid; dibasic/ monobasic phosphate, preferably dibasic/ monobasic phosphate at a molar concentration comprising from 0.005 to 0.1 molar. The preferred stabilizers used for the invention are selected from poly (oxy- 1, 2- ethanedyl) derivatives, and among these, more preferably poly (oxy- 1, 2- ethanedyl) mono- 9- octadecenoate, Polysorbate 80, which may be used in a range of concentrations from 0.01 to 1 mg/ml. Cryoprotectants useful for the invention are selected from carbohydrates such as saccharose or mannitol, surfactants such as glycerol, dimethylsulfoxide or Tween. Preferably, the cryoprotectant is a carbohydrate, more preferably saccharose in a range of concentrations from 20 to 100 mg/ml.

A further object of the present invention comprises the use of the recombinant human interferon-alpha mutein of the invention for manufacturing a medicament to be used in a therapeutic protocol including reduced therapeutic doses of IFN alpha 2b. It may be administered as a monotherapy or in the form of a combination therapy with ribavirin; as a monotherapy subcutaneously at a dose from 0.1 micrograms/kg to 2.0 micrograms/kg bodyweight/week. More preferably at a dose from 0.5 micrograms/kg to 1.0 micrograms/kg bodyweight/week. In the case of a combination therapy, a dose from 0.5 to 3.0 micrograms/kg bodyweight/week of the mutein of the invention is administered in combination with ribavirin capsules. More preferably, at doses from 1.0 to 2.0 micrograms/kg bodyweight/week of the mutein of the invention in combination with ribavirin capsules.

The following examples are included to assist further understanding of the present invention, but they should not be construed as a limitation of scope thereof. On the contrary, it should be clearly understood that those of skill in the art might envision other embodiments, modifications, and equivalents without departing from the spirit of the present invention and/or the scope of the appended claims.

## EXAMPLES

### Example 1: Isolation of human IFN-α2b gene from leucocyte genomic DNA

The hIFN-α2b gene was obtained by a PCR amplification reaction, using as a template genomic DNA from human peripheric blood leucocytes (Sambrook et al., 1989). For the PCR reaction, specific oligonucleotides hybridizing by both ends to the coding sequence of hIFN-α2b were used, designated IFNalphaF and IFNalphaR, as shown in Table I.

TABLE I: Oligonucleotides used for the construction of plasmids containing wild type rhIFN-α2b

| Oligonucleotide | Nucleotide sequence |
|---|---|
| IFNalphaF | 5' TAAC<u>GAATTC</u>ACATCTACAATGGCCTTGAC 3' *Eco*RI |
| IFNalphaR | 5' ATAG<u>TCTAGA</u>GTCTTTGAAATGGCAGATCA 3' *Xba*I |

A fragment of 673 pb was cloned into the commercial plasmid pGEM® T Easy Vector (Promega) following the protocol described by the manufacturer. The sequencing of this construct, designated pGEM- rhIFN- α2b*wt*, confirmed the identity of the cloned fragment, showing a 100% homology to the sequence published in GenBank for hIFN- α2b. Fig. 1 shows the nucleotide sequence that corresponds to hIFN- α2b cDNA and the amino acid sequence that corresponds to the encoded protein.

### Example 2: Selection of the amino acids to be modified in the rhIFN-α2b gene for obtaining new N-glycosylation sites

To select the amino acids to be mutated in order to artificially create new N- glycosilation sites, it was decided to carry out the least possible number of changes in the natural hIFN- α2b amino acid sequence. In this way, and keeping in mind the consensus sequence being sought (Asn- Xaa- Ser/Thr), two strategies were developed:

1- Identifying all serines and/or threonines in the natural hIFN-α2b amino acid sequence, the third position of the consensus sequence, and replacing the amino acid occupying the first position by asparagine.

2- Identifying all asparagines present in the natural hIFN-α2b sequence (first position of the consensus sequence) and mutating the amino acid in the third position by a serine or threonine.

Once those amino acids were identified, the resulting glycosylation sites of the corresponding mutations were screened for two basic aspects: high probability of glycosylation and conservation of biological activity of the protein.

The first aspect was evaluated in order to generate a high degree of occupation of the chosen sites. To that end, the following analysis was carried out:

a- Solvent Accessible Surface Area (ASA %) of the amino acid located in the first position of the consensus sequence (Asn or another amino acid mutated by Asn). This study provided information concerning the degree of exposed surface of the amino acid present in the protein structure that would bind the oligosaccharide chain. This parameter was calculated using the ASAview computer program (Ahmed, S., Gromiha, M., Fawereh, H., Sorci, A., BMC *Bioinformatics*, 2004, 5:51; http://www.netasa.org/asaview/).

b- Probability of glycosylation on the basis of the information from known glycoprotein databases. This parameter is determined using the NetNGlyc 1.0 Server program (www.cbs.dtu.dk/services/NetNGlyc/) created by the Center for Biological Sequence Analysis (CBS, Technical University of Denmark).

In addition, the selected mutations were screened for their capacity for avoiding loss of biological activity of the protein. This aspect was studied taking into account two features:

a- Proximity of the candidate glycosylation site to the molecular region involved in receptor binding. If this parameter is not taken into account, there might exist a high probability of generating glycoproteins having hydrocarbon chains interfering with the binding to the receptor, whereby biologically inactive forms of hIFN-α2b are obtained.

b- Solvent Accessible Surface Area (ASA%): the binding of oligosaccharides to hidden or internal residues of the protein might generate alterations of its tertiary structure, thereby decreasing biological activity.

Possible sites for a site-directed mutagenesis to obtain consensus sequence Asn-Xaa-Ser/Thr are: Leu9, Arg12, Leu66, Phe67, Lys70, Asp71, Phe84, Leu95, Glu113, Arg125, Met148, Ser150, Ser152, Leu153, Gln158 in secondary alpha-helix structures and Pro4, Thr6, Arg23, Leu26, Phe47, Ala50, Asp77, Gly104, Thr106, Lys134, Gln158, Leu161 located outside alpha-helix-type secondary structures.

Among possible sites for a site-directed mutagenesis to obtain consensus sequence Asn-Xaa-Ser/Thr, amino acids Pro4, Arg23, Lys70, and Asp77 were selected (shown in figure 1) and rhIFN-α2b constructs mutated by Asn in the above-indicated were obtained at said amino acid positions. These sites were selected using the above-mentioned tests and analysis.

**Example 3: Site-directed mutagenesis and cloning of mutated genes in expression vectors for eukaryotic cells**

The site-directed mutagenesis procedure for introducing N-glycosylation sites in the hIFN-α2b gene was performed using overlapping extension PCR, comprising basically 2 consecutive PCR reactions. The description of the technique is summarized in Fig. 2. Both PCR stages are carried out in a thermocycler, with 30 cycles consisting in the following stages: denaturation at 94° C for 1 minute, hybridization at 60° C for 30 seconds and elongation at 72° C for 1 minute.

The oligonucleotides used for the selected point mutations are described in Table II. In this way, nucleotide sequences corresponding to four mutated variants of hIFN-α2b were obtained, where a single N-glycosylation site was incorporated, designated rhIFN-α2bM4, rhIFN-α2bM23, rhIFN-α2bM70, and rhIFN-α2bM77. In these variants, the codons corresponding to amino acids Pro4, Arg23, Lys70, and Asp77 were substituted by a codon codifying for one Asn, respectively.

The resulting DNA fragments were digested with the EcoRI and XbaI restriction enzymes, and cloned into the pCI-neo expression vector, tus obtaining the constructs designated pCI-neo-rhIFN-α2bM4, pCI-neo-rhIFN-α2bM23, pCI-neo-rhIFN-α2bM70, and pCI-neo-rhIFN-α2bM77.

Table II: Oligonucleotides used for constructing plasmids containing different glycosylated variants

| Oligonucleotide | Nucleotide sequence |
|---|---|
| Mut4a | 5' GGGCTGTGATCTGAATCAAACCCACAGCCT 3' |

(continued)

| Oligonucleotide | Nucleotide sequence |
| --- | --- |
| Mut4b | 5' AGGCTGTGGGTTTGA__TT__CAGATCACAGCCC 3' |
| Mut23a | 5' GGCACAGATGAGGA__AT__ATCTCTCTTTTCTC 3' |
| Mut23b | 5'GAGAAAAGAGAGAT__A__TTCCTCATCTGTGCC 3' |
| Mut70a | 5' CTCTTCAGCACAAA__T__GACTCATCTGCTGCTTGG3' |
| Mut70b | 5' CCAAGCAGCAGATGAGTC__A__TTTGTGCTGAAGAG 3' |
| Mut77a | 5'CATCTGCTGCTTGGAATGAGACCCTCCTAGACA3' |
| Mut77b | 5'TGTCTAGGAGGGTCTCATT__CC__AAGCAGCAGATG 3' |

In order to obtain a mutant of rhIFN- $\alpha$2b with four N- glycosylation sites, designated pCI- neo- rhIFN- $\alpha$2bM4/23/70/77, an N- glycosylation site was added sequentially using the site- directed mutagenesis technique described above. In this way, a mutant with two N- glycosylation sites was constructed, using a molecule containing one N- glycosylation site as a template. Then a variant with three N- glycosylation sites was constructed using the molecule containing two N- glycosylation sites as a template. Finally, the mutant with four N- glycosylation sites was obtained, by a mutagenesis reaction with the molecule containing three N- glycosylation sites. In each step, each variant in the expression vector pCI- neo was cloned.

All DNA constructs containing the different N-glycosylated variants of rhIFN-$\alpha$2b were sequenced, which allowed to confirm the success of mutations.

## Example 4: Transient expression of N-glycosylated variants of human interferon-alpha in CHO.K1 cells (Chinese Hamster Ovary)

In order to evaluate gene integrity of each human interferon- alpha variant, its expression, level of cytokine glycosylation and specific activity of each variant, transfection of CHO.K1 cells was carried out by a lipofection technique using the plasmids pCI- neo- rhIFN- $\alpha$2bM4, pCI neo- rhIFN- $\alpha$2bM23, pCI- neo- rhIFN- a2bM70, pCI- neo- rhIFN- $\alpha$2bM77 and pCI- neo- rhIFN- $\alpha$2bM4/M23/M70/M77.

To this end, $3.10^5$ cells.ml$^{-1}$ CHO.K1 per well were seeded on a 6-well plate using a mammalian cell (MC) culture medium prepared from a 1:1 (V:V) mixture of D-MEM and Ham's F12 media supplemented with 2.441 g/l NaHCO$_3$, anhidrous D(+) glucose 6.6 mM, 1 mM sodium pyruvate, 7.8 mM glutamine, 0.13 mM tryptophan, 0.3 mM aspartic acid, 0.76 mM serine, and 50 $\mu$g/ml gentamycin sulphate. The medium was further supplemented with 5 % (V/V) bovine foetal serum (BFS). The next day, culture supernatants were removed and the cell layer was washed using MC medium. At the same time, different mixtures containing 10 $\mu$g of cathionic lipid (LipofectAMINE® 2000, Invitrogen) and 6 $\mu$g of the corresponding plasmid DNA diluted in MC medium were prepared. These preparations were incubated for 30 minutes at room temperature before addition to the cell culture. Then, the washing solution was removed from each well and the different liposome-containing solsutions were added and further incubated for 4 hours. Once incubated, the culture supernatant was removed and culture medium supplemented with 5 %(V/V) SFB was added. After 72 hours of incubation, the culture supernatant was harvested in order to evaluate the expression of glycosylated variants of rhIFN-$\alpha$2b.

Expression and glycosylation level of the different isoforms were screened using ELISA sandwich and Western Blot techniques, respectively.

## Example 5: Quantification of rhIFN $\alpha$2b using an ELISA sandwich assay

Flat bottom 96-well polystyrene plates were coated with 100 $\mu$l of a solution of 1 $\mu$g.ml$^{-1}$ mAb (100 ng per well) diluted in a 50 mM sodium carbonate/bicarbonate solution, pH 9.6. Plates were incubated for 1 hour at 37°C and overnight at 4°C. After washing 6 times with phosphate saline solution (PBS), 0.05 % (V/V) Tween 20, plates were blocked with 200 $\mu$l PBS with the addition of 1 % (W/V) bovine serum albumin (BSA). Then, 100 $\mu$l of 1:2 dilutions of non-glycosylated rhIFN-$\alpha$2b (standard protein) from 25 ng.ml$^{-1}$ to 0.39 ng.ml$^{-1}$ were added using PBS, 0.1 %(W/V) BSA, 0.05 % (V/V) Tween 20 (dilution solution), followed by incubation for 1 hour at 37°C. After washing, 100 $\mu$l of a solution of anti-rabbit rhIFN-$\alpha$2b polyclonal antibodies diluted 1:2.000 with dilution solution were added. Plates were incubated for 1 hour at 37°C. Finally, 100 $\mu$l of peroxidase-conjugated goat anti-rabbit immunoglobulin antibodies diluted 1:1.000 with dilution solution were added, followed by incubation for 1 hour at 37°C. After washing the plates, they were developed using as a substrate 0.12 volumes of H$_2$O$_2$ diluted in 50 mM phosphate citrate solution, pH 5.3, with the addition of O-phenylenediamine at a concentration of 3 mg.ml$^{-1}$. 100 $\mu$l of development solution were added to each well and plates were incubated in the dark at room temperature. After incubating the plates for 15 minutes, the color generated by the solution in each well was measured by absorbance at 450 nm using a microtiter plate reader. The results of the transient transfection experiments showed expression of recombinant rhIFN-α2b for all examined variants at 72-hours post-transfection.

### Example 6: Identification of rhIFN-α2b variants using a Western Blot assay

In a first step, a polyacrylamide gel electrophoresis procedure was carried out in the presence of sodium dodecylsulphate reagent (SDS-PAGE) and a sulphide bond reducing agent following substantially the method described by Laemmli (1970). To this end, samples were treated with a solution of 0.05 M Tris-HCl, 2 % (W/V) SDS, 10 % (V/V) glycerol, 5 % (V/V) β-mercaptoethanol, 0.05 % (W/V) bromophenol blue, pH 6.8. Samples were incubated at 100°C for 3 minutes and seeded directly on a stacking gel, having a 5 % (W/V) concentration of monomer/branching (acrylamide/ bisacrylamide). The separation gel was polymerized with acrylamide/bisacrylamide having a concentration of 15 % (P/V). Runs were performed at a constant voltage (200 mV) until the running front reached within 0.5 cm of the bottom of the separation gel. After electrophoresis, proteins were transferred to a nitrocellulose membrane (Bio-Rad). A standard protein transfer protocol was used employing 25 mM Tris, 192 mM glycine, 20 % (V/V) methanol, pH 8,3, as transfer solution. Transfer was carried out at a constant current intensity of 180 mA for 1 hour. After transfer was over, the success of the transfer was evaluated detecting the presence of proteins using a solution of 0.25 % (W/V) Ponceau Red in 15 % (V/V) glacial acetic acid and 40 % (V/V) methanol. Then, the dye was eluted by successive washes using Tris-Buffered Saline (TBS) and the membrane was blocked with a solution of TBS, 1 % (W/V) BSA. After three successive washes using TBS, the membrane was incubated in a solution of anti-rabbit rhIFN-α2b polyclonal antibodies diluted  1:1.000 with dilution solution (0.1 % (P/V) TBS, BSA). After the corresponding washes, the membrane was incubated in a 1: 2,000 solution of goat anti-rabbit immunoglobulin antibodies conjugated with peroxydase. All incubations were carried out with stirring for 1 hour at room temperature. Finally, the membrane was developed by a chemoluminiscent reaction using the ECL Plus Western Blotting Reagent commercial kit (GE Healthcare). Luminescence emission was detected by exposure of the membrane to photographic films for a variable time period. Film development was carried out manually employing a conventional fixation and photographic developing method.

All variants containing a single site susceptible to N-glycosylation exhibited a mixture of IFNα muteins having different molecular mass, corresponding to non-glycosylated, O-glycosylated, and N,O-glycosylated fractions, in varying proportions according to the examined mutant. In the particular case of rhIFN-α2bM70 mutein, expression of muteins of higher molecular mass as comparted to the remaining muteins incorporating a single site susceptible to N-glycosylation was observed. Said isoforms would correspond to molecules exhibiting a higher content of glycosidic structures. For rhIFN-α2bM4/M23/M70/M77 mutein; the assay showed a variable glycosylation level with disappearance of the O-glycosylated isoform and a reduced proportion of of those corresponding to a low degree of occupation. In addition, a greater concentration of isoforms with a high level of occupation having a molecular mass ranging from 21 and 45 kDa was observed.

In all cases (mutants carrying a single site or 4 sites susceptible to N-glycosylation) the presence of oligosaccharides linked by N-type bonds was confirmed by a specific N-deglycosylation treatment using the PNGase F enzyme (Asparagine amidase, Biolabs). In this manner, all muteins showed matching protein bands corresponding to the O-glycosylated variant, thus confirming the presence of hydrocarbon chains linked through an N-glycosidic bond to the thus incorporated sites. Figure 3 illustrates the results obtained.

### Example 7: Evaluation of the stability of N-glycosylated variants of hIFN-a2b at positions Arg23, Asn45, Lys70 and Asn93, in the presence of the PNGase F enzyme (N-glycanase)

The different hIFN-α2b molecules, mutated individually at amino acid positions Arg23, Leu47, Lys70, and Leu95 so as to obtain glycosylation sites at positions Arg23, Asn45, Lys70, and Asn93, were tested for stability in the presence of the PNGase F enzyme responsible for the release of oligosaccharides bound through an N-glycosidic bond. To this end, 350 μl of culture supernatant for each of the above-mentioned mutants were treated with 250 U of PGNase F (Biolabs) and incubated at 37°C. The deglycosylation reaction was inhibited at different time intervals (30, 60, 90, 120, 150, 180, and 300 minutes) by taking 45 μl aliquotes from said mixture and incubating them with 15 μl of a 0.05 M Tris-HCl solution, 2 % (W/V) SDS, 10 % (V/V) glycerol, 5 % (V/V) β-mercaptoethanol, 0.05 % (W/V) bromophenol blue, pH 6.8, for 5 minutes at 100°C. Once the deglycosylation reaction was completed, all samples were analyzed by a reducing SDS-PAGE technique and then a Western blot analysis substantially following the steps as indicated in example 6.

The percentage of N, O- glycosylated, O- glycosylated, and non- glycosylated isoforms in the samples was estimated by densitometry and then their proportion was calculated using the commercial software ImageMaster TotalLab V1.11 (GE Healthcare, Sweden) .

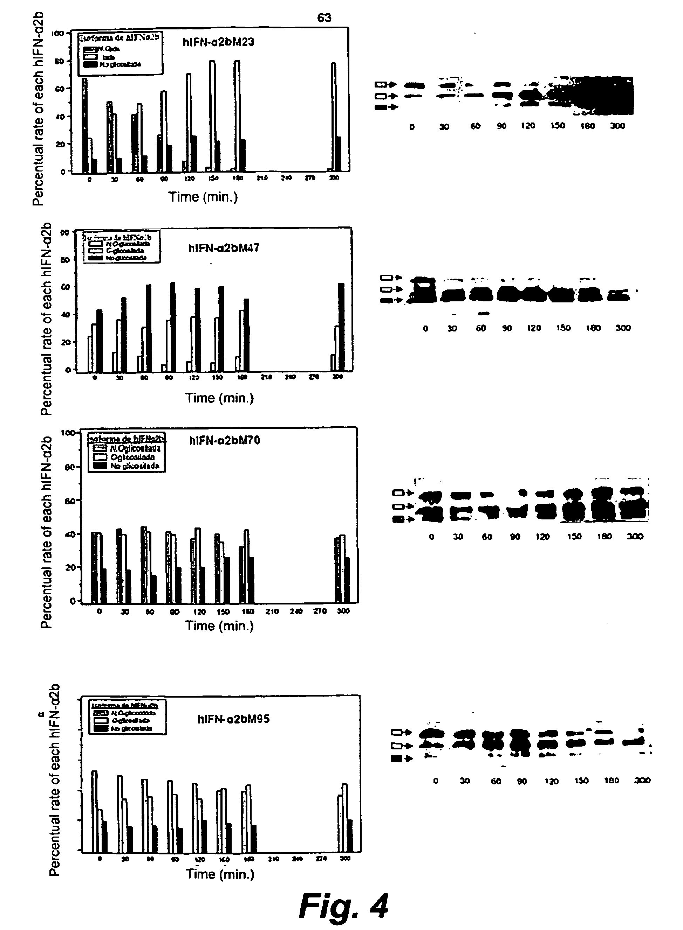

Fig. 4 summarizes the results of enzymatic deglycosylation of 4 mutated hIFN-α2b. Mutant IFN-α2bM23 showed complete removal of the N-glycosydic bond after 150 minutes of incubation with the enzyme, whereas mutant IFN-$\alpha$2bM47 reduced its degree of N-glycosylation by 50 % after 30 minutes of enzymatic treatment. In addition, mutants IFN-$\alpha$2bM70 and IFN-$\alpha$2bM95 conserved approximately 40 % of N,O-glycosylated variants by the end of the experiment, as compared to total IFN forms present in each sample.

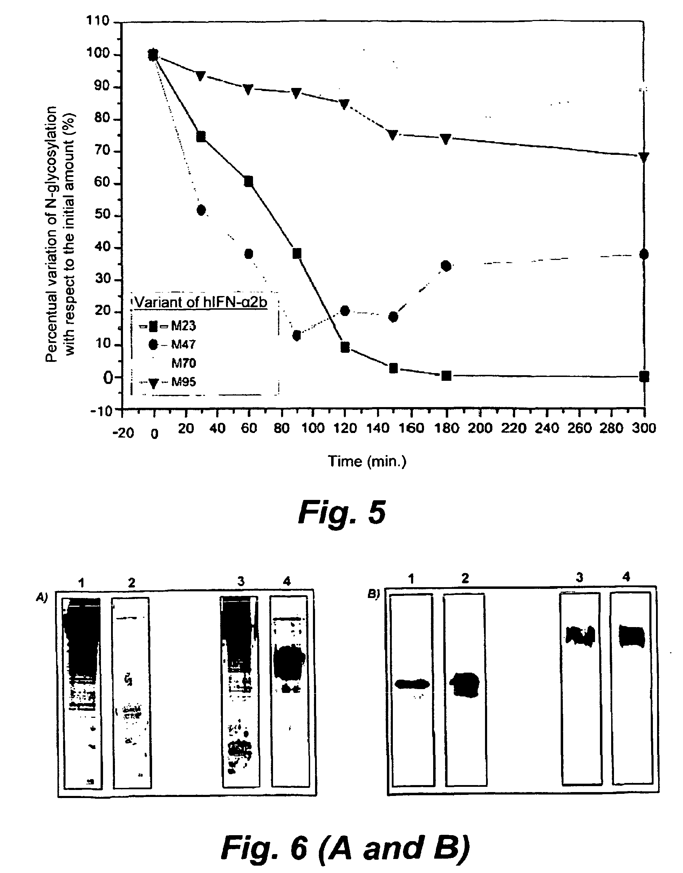

Fig. 5 shows the percent variation of N-glycosylation as a function of time expressed as percentage of glycosylation with respect to the amount present at the beginning of the enzymatic deglycosylation procedure. Notably, the greatest stability is visualized for the IFN-$\alpha$2bM70 and IFN-$\alpha$2bM95 variants that conserved a high ratio of said isoform (90% and 70%, respectively) after 300 minutes of reaction.

When analyzing the molecular structure of hIFN-$\alpha$2b, it may be observed that amino acid position Arg23 is located at the beginning of a connecting loop of two $\alpha$-helix structures and amino acid position Phe47 is located in the central region of another connecting loop, which confers greater susceptibility to the action of a deglycosylating enzyme. On the other hand, positions Arg70 and Leu95 form part of an $\alpha$-helix. This type of structure might confer a greater resistance to both sites and as a consequence a superior stability to the action of N-glycanases.

**Example 8: Evaluation of *in vitro* antiviral biological activity of mutated variants of rhIFN-$\alpha$2b**

Biological activity of the different hIFN-$\alpha$2b variants was determined by measuring the antiviral effect of the same on cultures of MDBK cells (Madin-Darby Bovine Kidney, ATCC CCL-22) infected with vesicular stomatitis virus (VSV), Indiana strain (ATCC VR-158). To this end, flat bottom 96-well plates were seeded with 100 $\mu$l of a cellular suspension containing 250,000 cell.ml$^{-1}$ using MEM culture medium supplemented with 2 mM L-glutamine, 2.2 mg.ml$^{-1}$ sodium bicarbonate, 50 $\mu$g.ml$^{-1}$ gentamycin, and 10 %(V/V) bovine foetal serum (growth medium).

Plates were incubated overnight at 37°C and 5% $CO_2$, saturated humid atmosphere. The next day, culture supernatant was removed and 100 $\mu$l of consecutive 2-fold dilutions of non-glycosylated rhIFN-$\alpha$2b (standard) from 20 U.ml$^{-1}$ to 0.156 U.ml$^{-1}$ were added and the corresponding test samples at appropriate dilutions. All dilutions were carried out in a culture medium identical to the growth medium with a concentration of 2% (V/V) SFB (assay medium). Plates were incubated in an oven for 6 hours. After this, supernatant was removed and plates were washed using assay medium. Finally, 100 $\mu$l of VSV virus suspension prepared in assay medium were added. A viral dilution capable of generating 100 % cytopathic action after 20-24 hours was used as working dilution. Plates were incubated overnight at 37°C and 5% $CO_2$, saturated humid atmosphere. The next day, when a cytopathic action of about 100 % in control wells in absence of IFN was observed, culture supernatant was removed by inversion of the plates and development thereof was carried out using 50 $\mu$l of 0.75 %(W/V) crystal violet solution in 40 %(V/V) methanol. Plates were incubated at 37°C for 15 minutes. The dye was removed and plates were washed with distilled water until absence of dye was verified in the washes. After blotting the plates, 250 $\mu$l of 20 %(V/V) acetic acid solution were added. Plates were homogeneized until each well showed a uniform color, and finally color absorbance was determined by spectrophotometric measuring at $\lambda$=540 nm.

The specific biological activity parameter (ABE) was determined for each molecule (Table III). rhIFN-$\alpha$2bM4, rhIFN-$\alpha$2bM23, and rhIFN-$\alpha$2bM77 mutants showed variable levels of specific activity within a range from 160 to 290 U.ng$^{-1}$. In this manner, incorporation of mutations in the protein sequence and/or the presence of N-type hydrocarbon chains did not alter significantly the activity of said variants as compared to the specific biological activity of non-glycosylated rhIFN-$\alpha$2b (200 U$^{-1}$). In the case of rhIFN-$\alpha$2bM70 mutein a 48% decrease of the above-mentioned parameter was evident. However, considering that muteins IFN-$\alpha$2bM4, IFN-$\alpha$2bM23 and IFN-$\alpha$2bM77 conserved their specific biological activity, and a higher level of glycosylation of mutein rhIFN-$\alpha$2bM70, the corresponding mutein carrying the 4 N-glycosylation susceptible sites indicated previously was obtained. When assessing its specific biological activity (26 U.ng$^{-1}$), we observed an 87% decrease as compared to the non-glycosylated molecule.

Table III: Evaluation of different glycosylated muteins of rhIFN-$\alpha$2b

| Mutein of rhIFN-$\alpha$2b | Concentration(U.ng$^{-1}$) | ABE (ng.ml$^{-1}$) |
|---|---|---|
| IFN-$\alpha$2bM4 | 186 | 265 |
| IFN-$\alpha$2bM23 | 143 | 287 |
| IFN-$\alpha$2bM70 | 221 | 105 |
| IFN-$\alpha$2bM77 | 153 | 164 |
| IFN-$\alpha$2bM4/23/70/77 | 109 | 26 |

**Example 9: Obtention of mutated rhIFN-α2b-producing stable cell lines**

CHO.K1 cells were transiently transfected using different constructs essentially following the stages as described in Example 4 for obtaining N- glycosylated rhIFN- α2b- producing stable cell lines. Then, the cultures were subjected to selection pressure using the antibiotic Neomycin (200 μg.ml$^{-1}$) from 72 to 168 hours post- transfection. After two weeks of culture in the presence of the antibiotic, cytokine expression levels were quantified by a sandwich ELISA assay and producing lines (CHO pCI- neo- rhIFN- α2bM4, CHO pCI- neo- rhIFN- α2bM23, CHO pCI- neo- rhIFN- α2bM70, CHO pCI- neo- rhIFN- α2bM77, and CHO pCI- neo- rhIFN- α2bM4/23/70/77) were kept in liquid nitrogen. Then, the cell lines were cloned using limiting dilution methods. Table IV shows details of selected clones and their specific productivity in the stationary phase. The clones of each cell line showing higher productivity are shown in bold. They were used later in the production phase of each mutated rhIFN- α2b variant.

Table IV: Cloning of producer lines and evaluation of specific productivity of the clones thus obtained

| Line | Clon | Specific productivity (ng. $10^6$ cell$^{-1}$.day$^{-1}$) |
|---|---|---|
| CHO pCI-neo-IFN-α2bM4 | M41A6 | 20.52 |
| | M42E5 | 27.01 |
| | **M42F8** | **45.43** |
| | M42G5 | 16.40 |
| CHO pCI-neo-IFN-α2bM23 | M231 F2 | 46.67 |
| | **M231D7** | **106.93** |
| CHO pCI-neo-IFN-α2bM70 | **M701E3** | **42.55** |
| | M702B2 | 6.67 |
| CHO pCI-neo-IFN-α2bM77 | M771A6 | 36.95 |
| | **M771C6** | **76.29** |
| | M772B1 | 47.27 |
| | M772D4 | 54.37 |
| | M772E7 | 44.33 |
| | M772G8 | 36.30 |
| CHO pCI-neo-IFN-α2bM4/23/7077 | L1A8 | 19.16 |
| | L1B8 | 21.26 |
| | L2A8 | 11.55 |
| | L2C8 | 39.72 |
| | **3D2F5** | **46.50** |
| | 3D2C8 | 12.28 |
| | 3D2G8 | 6.65 |
| | 7D1H6 | 16.86 |
| | 7D1 F7 | 9.92 |
| | 7D2H3 | 5.91 |
| | 7D2A5 | 6.99 |
| | 7D2H7 | 5.46 |
| | 7D2F8 | 2.32 |
| | 7D1H6 | 16.86 |
| | 7D1F7 | 9.92 |
| | 7D2H3 | 5.91 |

**Example 10: Production of mutated rhIFN-α2b by culturing the selected clones in adherence conditions**

In a first step, the specific productivity of rhIFN-α2b during different cell culture phases was determined, indicating the clones showing greater specific productivity of the cytokine during the stationary phase.

Secondly, different concentrations of SFB were assessed as culture medium supplements to reduce protein content in supernatants and facilitate further purification of rhIFN- α2b variants. To this end, clones were cultured using culture medium (MC) supplemented with 5% (V/V) SFB and, when they reached a stationary phase, the conditioned supernatant was replaced by fresh culture medium containing different serum concentrations: 5%, 1%, 0.5%, and 0.1% (V/V) . After 24 hours of culture, a drastic drop of specific productivity was observed when changing SFB concentration from 5% to 1% (V/V) . However, specific productivity using from 1% to 0.5% (V/V) SFB concentrations was similar, and therefore the latter serum concentration was used for carrying out the production. By this, a 10- fold reduction of protein content was achieved in the samples to be purified.

Finally, an experiment for determining the number of culture medium changes and optimum time- interval between them was carried out in order to obtain the greatest recombinant protein mass as possible. A total of eight harvests were carried out, and their corresponding changes of culture medium, every 24 hours and four harvests every 48 hours in stationary phase cultures. It was found that total accumulated mass for rhIFN- α2b was similar in both experiments, and a higher concentration of cytokine in supernatants harvested every 48 hours was obtained.

In all the above-mentioned experiments, the quality of the proteins thus produced was evaluated and it was shown that, irrespective of the assay conditions, the specific biological activity and isoform molecular mass profiles for the different molecules were conserved.

The different rhIFN-α2b variants were obtained using 500 cm$^2$ culture flasks. To this end, approximately 2.10$^5$ cell.ml$^{-1}$ were seeded using MC culture medium supplemented with 5% (V/V) SFB. When the culture reached the stationary phase of growth, sequential changes every 48 and 72 hours were performed using MC culture medium supplemented with 0.5% (V/V) SFB. Culture supernatants were centrifuged at 3,000 r.p.m for 10 minutes and stored at -20°C for further purification of the corresponding variants.

**Example 11: Purification of recombinant human interferon-alpha mutein of the invention from culture supernatants.**

The different mutated variants of rhIFN-α2b were purified by an immunoaffinity chromatography method, using the mAb selected as capturing antibody as a ligand in a sandwich ELISA assay.

In the first place, 10 female BALB/c mice of 2 months of age were used for the *in vivo* production of mAb in ascitic liquid. To this end, 0.5 ml of 2, 6, 10, 14- tetramethyldecanoic acid (Pristane®, Sigma) were inoculated intraperitonially in each mouse and after 10 days 2.10$^6$ hybridoma cells per animal resuspended in 0.5 ml of PBS were inoculated by the same route. The ascitic liquid was collected by intraperitoneal puncture using a 1.2 mm gauge needle, from the first week after inoculation of hybridomas. The concentration of murine immunoglobulins in the ascitic fluid was determined using a sandwich ELISA assay.

Purification of mAb was carried out by affinity chromatography using a Sepharose High Trap protein A 5 ml column (GE Healthcare), following the method described by Harlow and Lane (1998). Pure immunoglobulin concentration was calculated by spectrophotometric readings at 280 nm using a percent extinction coefficient $\left( E_{1cm}^{1\%} \right)$ of 12.5.

Multiplying $10 \times \left( E_{1cm}^{1\%} \right)^{-1}$ by optical density readings is equal to mAb concentration expressed in mg.ml$^{-1}$.

The affinity ligand was coupled to a Sepharose 4B matrix activated with cyanogen bromide following a standard protocol (GE Healthcare). The percentage of mAb coupling to the resin was of 98,3% and theorical matrix capacity was of 186 μg of non-glycosylated rhIFN-α2b per ml of gel.

The following purification protocol was used for rhIFN-α2b muteins with one N-glycosylation site and with four N-glycosylation sites. Culture supernatants containing cytokines were worked up by addition of Triton X-100 until a concentration of 0.3% (V/V) was achieved. The worked-up samples were loaded onto a column previously adjusted using a solution of 0.1 M Tris, 0,3% (V/V) (pH 7.5) Triton X-100 at a rate of 0.5 ml.min$^{-1}$. Then, it was washed with 5 column volumes using each of the following solutions:

1- solution of 0.5 M NaCl, 0.2% (V/V) Triton X-100 in 0.025 M Tris (pH 7.5).
2-solution of 0.15 M NaCl in water (pH 5.0).

Elution of rhIFN-α2b was assessed using three different solutions:

1- solution of 0.15 M NaCl in 0,2 M acetic acid (pH 3.0)
2- solution of 0.1 M glycine (pH 2.5)
3- solution of 0.1 M glycine (pH 2.0).

2 ml fractions were collected. Each fraction was neutralized by addition of a solution of 1 M Tris (pH 9.0). The matrix was stored in 0.02 % (W/V) azide solution in 0.025 M Tris (pH 7.5).
The presence of rhIFN-α2b was evaluated in all the fractions by a sandwich ELISA assay, achieving a higher recovery percent (ranging from 95% to 100 %) by using a 0.1 M glycine solution (pH 2.0).
Purification efficiency was studied by SDS-PAGE analyses under reductive conditions with additional silver staining and Western blot assays (Figure 6). Purity percent, estimated by band densitometry, was of 85 %. In addition, Western blot assays showed that, for both rhIFN-α2b muteins, the isoform profile for the purified molecule was very similar to that of the cytokine present in culture supernatants.

**Example 12- Evaluation of pharmacokinetic parameters of variants having a single N-glycosylation site designated rhIFN-α2bM23 and rhIFN-α2bM70 in experimental animals using a subcutaneous inoculation route**

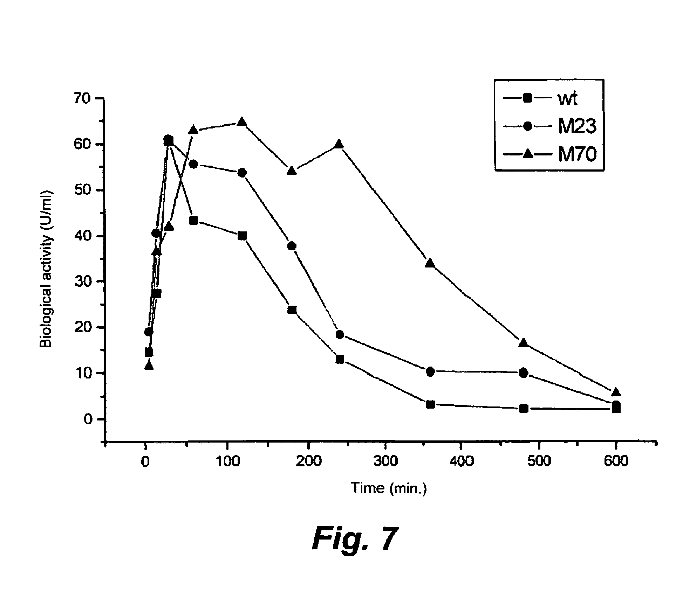

Pharmacokinetic properties of variants having an N- glycosylation site located outside α- helix regions (rhIFN-α2bM23) and forming part of an α- helix (rhIFN- α2bM70) were evaluated using a subcutaneous inoculation route. To that end, female Wistar rats, of two months of age, weighting 200 g on average, were inoculated with the indicated IFN variants using a single dose of $5 \times 10^5$ U.Kg$^{-1}$/ body weight of the animal. Then, blood samples were collected by puncture of the retroorbital vein using heparinized capillary tubes at different post- injection times and the presence of the cytokine was determined quantifying its volumetric biological activity. Data thus obtained were used to plot biological activity versus  time (Fig. 7) . The same experiment was carried out using the IFN wild type (rhIFN- α2bwt) molecule.
The area under the biological activity curves (AUC), calculated as a function of time corresponding to the glycosylated rhIFN-α2bM70 mutein showed an 1.63-fold increase as compared to the rhIFN-α2bM23 variant.

**Example 13: Evaluation of pharmacokinetic parameters of different rhIFN-a2b variants in animal models**

13-a)- Intravenous inoculation route

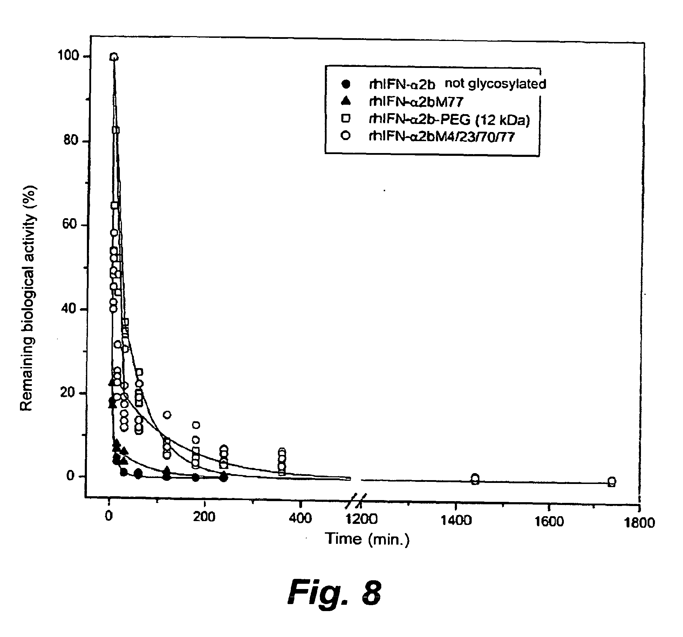

In order to evaluate the influence of glycosidic moieties on the pharmacokinetic parameters of glycosylated rhIFN- α2b variants, the rhIFN- α2bM77 variant (representing molecules having a single N- glycosylation site located outside an alpha- helix structure) and the rhIFN- α2bM4/23/70/77 mutein were selected. Studies were carried out by comparison to the rhIFN- α2b molecule produced in bacteria (non- glycosylated) and to versions covalently conjugated to a molecule of 12 kDa polyethylenglycol (PEG) (rhIFN- α2b- PEG 12kDa; Schering Plough) . To that end, female Wistar rats, of two months of age, having an average weight of 200 g, were inoculated with a single dose of 52.6 pmol of each IFN variant per animal using, in the first place, an intravenous administration route (tail vein) . In this assay, batches of 4 animals per each variant were used. Then, blood samples were collected by puncture of the retroorbital vein using heparinized capillary tubes at different post- injection times. They were used to determine the presence of the cytokine quantifying its volumetric biological activity (AB) by the above- mentioned biological activity *in vitro* assay. Data thus obtained were used to calculate the percent of remnant biological activity in each sample as compared to biological activity units of IFN administered to each rat and said percentage was plotted versus time from inoculation (Fig. 8) .
The behavior of the different molecules was appropriately adjusted to a bicompartimental model, according to the following equation:

$$C = A \cdot e^{-t/t1} + B \cdot e^{-t/t2},$$

wherein C is the fraction of circulating remnant drug at time t, A and t1 are initial phase parameters reflecting protein distribution to extravascular body  fluids, whereas B and t2 are parameters characterizing the terminal phase of drug removal.
Thus, a regression adjustment was carried out for each curve, and pharmacokinetic parameters as indicated in Table V were determined.

Table V. Evaluation of pharmacokinetic parameters of different rhIFN-α2b variants.

| Variant | t1 (minute) | t2 (minute) | Plasmatic *Clearance* (ml.min$^{-1}$) |
|---|---|---|---|
| non-glycosylated rhIFN-α2b | 2.3 $\pm$ 0.1 | 14.1 $\pm$ 2.8 | 3,21 |
| rhIFN-α2b M77 | 2.4 $\pm$ 0.2 | 62.0 $\pm$ 0.7 | 2,33 |
| rhIFN-α2b M4/23/70/77 | 4.7 $\pm$ 0.3 | 125.8 $\pm$ 16.4 | 0,36 |
| rhIFN-α2b-PEG (12kDa) | 4.3 $\pm$ 1.0 | 59.6 $\pm$ 6.0 | 0,37 |

As may be seen from this Table, half-life times of the initial phase (proportional to t1) of non-glycosylated rhIFN-α2b and rhIFN-α2bM77 molecules are similar to each other, but lower as compared to values calculated for the rhIFN-α2bM4/23/70/77 and rhIFN-α2b-PEG variants. In addition, half-life times corresponding to the removal phase was higher for the rhIFN-α2bM4/23/70/77 muteins.

Taking into account non-glycosylated and glycosylated IFN variants, a reduction of plasmatic clearance was observed as hydrocarbon content increased. In the particular case of the rhIFN-α2bM4/23/70/77 mutein, a 9-fold reduction of said parameter was visualized as compared to the non-modified rhIFN-α2b molecule. In additioin, no significant differences were observed when calculating plasmatic clearance corresponding to rhIFN-α2bM4/23/70/77 and rhIFN-α2b-PEG molecules.

13 b)-Subcutaneous inoculation route

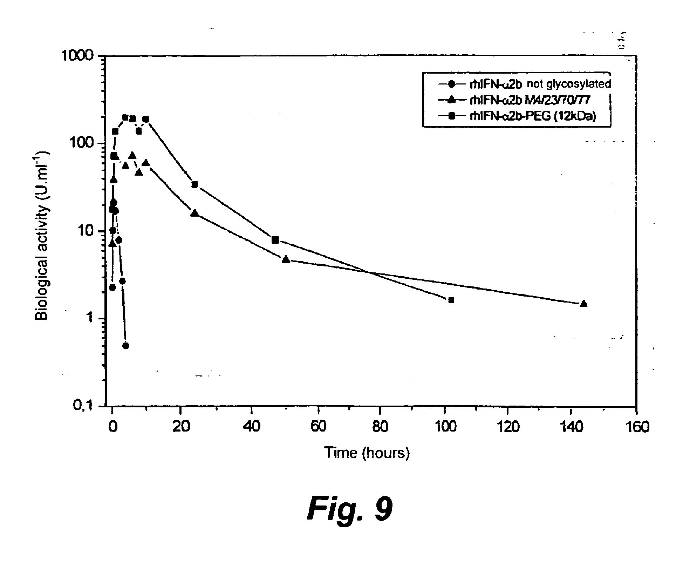

In addition, pharmacokinetic properties of the mutein having four N- glycosylation sites (IFN- α2bM4/23/70/77) were evaluated using a subcutaneous inoculation route. To that end, female Wistar rats, of two months of age, having an average weight of 200 g, were inoculated with the indicated IFN variant using a single dose of 5 x 10$^5$ U.Kg$^{-1}$ of body weight. At the same time, animals were inoculated with identical doses of non- glycosylated rhIFN- α2b or rhIFN- α2b-PEG 12kDa. Then, blood samples were collected by puncture of the retroorbital vein using heparinized capillary tubes at different post- injection times. They were used to determine the presence of the cytokine by quantifying its volumetric biological activity by the above- mentioned *in vitro* assay of biological activity. The values thus obtained were used to plot biological activity in each sample as a function of time (Fig. 9) .

The area under the biological activity curve (AUC) calculated as a function of time corresponding to the N-glycosylated rhIFN-α2bM4/23/70/77 mutein showed a 37-fold increase as compared to the non-glycosylated cytokine variant. At the same time, glycosylated cytokine reached a maximum biological activity concentration from the first hour of post-injection, remaining approximately constant for 10 hours. Said maximum concentration was higher than that achieved by the non-glycosylated variant, evidenced as a peak concentration after 30 minutes of post-inoculation. Finally, biological activity levels of the latter variant became non-detectable after 4 hours of post-injection.

## Claims

1. A recombinant human interferon-alpha mutein having at least one amino acid substitution in the sequence of the human natural interferon-alpha, resulting in the consensus sequence Asn-Xaa-Ser/Thr where the Asn residue is capable of being N-glycosylated, at position Lys70 with an Asn residue (Lys70Asn).

2. A recombinant human interferon-alpha mutein according to claim 1, **characterized in that** said mutein contains a glycosylation site at position Lys70 and further an N-glycosylation site at a position selected from the group consisting of positions Pro4, Arg23, and Asp77.

3. A recombinant human interferon-alpha mutein according to claim 1, **characterized in that** said mutein has three N-glycosylation sites, one N-glycosylation site at position Lys70 and two N-glycosylation sites selected from the group consisting of positions Pro4, Arg23, and Asp77.

4. A recombinant human interferon-alpha mutein according to claim 1, **characterized in that** said mutein contains four N-glycosylation sites selected from the group consisting of amino acid positions Pro4, Arg23, Lys70, and Asp77.

5. A recombinant human interferon-alpha mutein according to claim 1, **characterized in that** said mutein is stable in the presence of N-glycanase at least of 70% of its initial concentration, after 300 minutes.

**6.** A gene encoding a recombinant human interferon-alpha mutein, wherein said gene encodes an amino acid sequence having at least one amino acid substitution, resulting in the consensus sequence Asn-Xaa-Ser/Thr where the Asn residue is capable of being N-glycosylated, at position Lys70 with an Asn residue (Lys70Asn).

**7.** A gene encoding a recombinant human interferon-alpha mutein according to claim 6, wherein said gene encodes an amino acid sequence having a glycosylation site at position Lys70 and further an N-glycosylation site at a position selected from the group consisting of positions Pro4, Arg23, and Asp77.

**8.** A gene encoding a recombinant human interferon-alpha mutein according to claim 6, wherein said gene encodes an amino acid sequence having three N-glycosylation sites, one N-glycosylation site at position Lys70 and two N-glycosylation sites selected from the group consisting of positions Pro4, Arg23, and Asp77.

**9.** A gene encoding a recombinant human interferon-alpha mutein according to claim 6, wherein said gene encodes an amino acid sequence having four N-glycosylation sites selected from the group consisting of amino acid positions Pro4, Arg23, Lys70, and Asp77.

**10.** A pharmaceutical formulation comprising at least a recombinant human interferon-alpha mutein according to claim 1.

**11.** A pharmaceutical formulation comprising at least a recombinant human interferon-alpha mutein according to claim 2.

**12.** A pharmaceutical formulation comprising at least a recombinant human interferon-alpha mutein according to claim 3.

**13.** A pharmaceutical formulation comprising at least a recombinant human interferon-alpha mutein according to claim 4.

**14.** A pharmaceutical formulation comprising at least a recombinant human interferon-alpha mutein according to claim 5.

**15.** A pharmaceutical formulation according to claims 10 to 14, further comprising pharmacologically acceptable excipients.

**16.** A pharmaceutical formulation according to claims 10 to 14, wherein said formulation comes in liquid solution, solid, emulsion, cream, gel and particles.

**17.** A use of the mutein according to any one of claims 1 to 6 to prepare a medicament for treatment of disease susceptible of being treated with interferon alpha 2b, wherein said disease is selected from the group consisting of melanoma, chronic hepatitis C, acute and chronic hepatitis B, acute and chronic non-A, non-B hepatitis, Kaposi's sarcoma, multiple sclerosis, genital warts, leukemia, viral infections.

**18.** Uses according to claim 17 wherein said medicament is administered by a route of administration selected from the group consisting of subcutaneous, parenteral, oral, sublingual, intranasal, topic routes.

**Patentansprüche**

**1.** Rekombinantes Mutein des menschlichen Interferon-Alpha, welches zumindest eine Aminosäuresubstitution in der Position Lys70 mit einem Asn-Rest (Lys70Asn) in der Sequenz des menschlichen natürlichen Interferon-Alpha aufweist, wobei die Konsensussequenz Asn-Xaa-Ser/Thr in welcher der Asn-Rest N-glycosiliert sein kann, erhalten wird.

**2.** Rekombinantes Mutein des menschlichen Interferon-Alpha nach Anspruch 1, **dadurch gekennzeichnet, dass** das genannte Mutein einen Glycosylierungsort in der Position Lys70 und zusätzlich einen N-Glycosylierungsort in einer Position ausgewählt aus der Gruppe bestehend aus den Positionen Pro4, Arg23 und Asp77 enthält.

**3.** Rekombinantes Mutein des menschlichen Interferon-Alpha nach Anspruch 1, **dadurch gekennzeichnet, dass** das genannte Mutein drei N-Glycosylierungsorte aufweist: einen N-Glycosylierungsort in der Position Lys70 und zwei N-Glycosylierungsorte ausgewählt aus der Gruppe bestehend aus den Positionen Pro4, Arg23 und Asp77.

**4.** Rekombinantes Mutein des menschlichen Interferon-Alpha nach Anspruch 1, **dadurch gekennzeichnet, dass** das genannte Mutein vier N-Glycosylierungsorte ausgewählt aus der Gruppe bestehend aus den Aminosäurepositionen Pro4, Arg23, Lys70 und Asp77 enthält.

**5.** Rekombinantes Mutein des menschlichen Interferon-Alpha nach Anspruch 1, **dadurch gekennzeichnet, dass** nach 300 Minuten das genannte Mutein in Gegenwart von N-Glycanase zumindest zu 70% in Bezug auf seine anfängliche Konzentration stabil ist.

**6.** Gen codierend für ein rekombinantes Mutein des menschlichen Interferon-Alpha, wobei das genannte Gen für eine Aminosäuresequenz codiert, welche zumindest eine Aminosäuresubstitution in der Position Lys70 mit einem Asn-Rest (Lys70Asn) aufweist, wobei die Konsensussequenz Asn-Xaa-Ser/Thr in welcher der Asn-Rest N-glycosiliert sein kann, erhalten wird.

**7.** Gen codierend für ein rekombinantes Mutein des menschlichen Interferon-Alpha nach Anspruch 6, wobei das genannte Gen für eine Aminosäuresequenz codiert, welche einen Glycosylierungsort in der Position Lys70 und zusätzlich einen N-Glycosylierungsort in einer Position ausgewählt aus der Gruppe bestehend aus den Positionen Pro4, Arg23 und Asp77 aufweist.

**8.** Gen codierend für ein rekombinantes Mutein des menschlichen Interferon-Alpha nach Anspruch 6, wobei das genannte Gen für eine Aminosäuresequenz codiert, welche drei N-Glycosylierungsorte aufweist: einen N-Glycosylierungsort in der Position Lys70 und zwei N-Glycosylierungsorte ausgewählt aus der Gruppe bestehend aus den Positionen Pro4, Arg23 und Asp77.

**9.** Gen codierend für ein rekombinantes Mutein des menschlichen Interferon-Alpha nach Anspruch 6, wobei das genannte Gen für eine Aminosäuresequenz codiert, welche vier N-Glycosylierungsorte ausgewählt aus der Gruppe bestehend aus den Aminosäurepositionen Pro4, Arg23, Lys70 und Asp77 aufweist.

**10.** Arzneimittelformulierung umfassend zumindest ein rekombinantes Mutein des menschlichen Interferon-Alpha nach Anspruch 1.

**11.** Arzneimittelformulierung umfassend zumindest ein rekombinantes Mutein des menschlichen Interferon-Alpha nach Anspruch 2.

**12.** Arzneimittelformulierung umfassend zumindest ein rekombinantes Mutein des menschlichen Interferon-Alpha nach Anspruch 3.

**13.** Arzneimittelformulierung umfassend zumindest ein rekombinantes Mutein des menschlichen Interferon-Alpha nach Anspruch 4.

**14.** Arzneimittelformulierung umfassend zumindest ein rekombinantes Mutein des menschlichen Interferon-Alpha nach Anspruch 5.

**15.** Arzneimittelformulierung nach den Ansprüchen 10 bis 14, welche zusätzlich pharmakologische annehmbare Hilfsstoffe umfasst.

**16.** Arzneimittelformulierung nach den Ansprüchen 10 bis 14, wobei die genannte Formulierung als flüssige Lösung, Feststoff, Emulsion, Creme, Gel und Teilchen erhältlich ist.

**17.** Verwendung des Muteins nach einem der Ansprüche 1 bis 6, zum Zubereiten eines Medikamentes für die Behandlung einer Krankheit welche mit Interferon-Alpha 2b behandelt werden kann, wobei die genannte Krankheit aus der Gruppe bestehend aus Melanom, chronischer Hepatitis C, akuter und chronischer Hepatitis B, akuter und chronischer Non-A Non-B Hepatitis, Kaposi-Sarkom, multipler Sklerose, Genitalwarzen, Leukämie und Virusinfektionen ausgewählt wird.

**18.** Verwendung nach Anspruch 17, wobei das genannte Medikament durch einen Verabreichungsweg ausgewählt aus der Gruppe bestehend aus den subkutanen, parenteralen, oralen, sublingualen, intranasalen, topischen Wegen verabreicht wird.

**Revendications**

1. Mutéine de l'interféron alpha humain recombinant ayant au moins une substitution d'acide aminé dans la séquence de l'interféron alpha naturel humain, donnant comme résultat la séquence consensus Asn-Xaa-Ser/Thr où le résidu Asn peut être N-glycosilé, dans la position Lys70 avec un résidu Asn (Lys70Asn).

2. Mutéine de l'interféron alpha humain recombinant selon la revendication 1, **caractérisée en ce que** ladite mutéine contient un site de glycosylation dans la position Lys70 et en outre un site de N-glycosylation dans une position choisie parmi le groupe constitué par les positions Pro4, Arg23 et Asp77.

3. Mutéine de l'interféron alpha humain recombinant selon la revendication 1, **caractérisée en ce que** ladite mutéine possède trois sites de N-glycosylation, un site de N-glycosylation dans la position Lys70, et deux sites de N-glycosylation choisis parmi le groupe constitué par les positions Pro4, Arg23 et Asp77.

4. Mutéine de l'interféron alpha humain recombinant selon la revendication 1, **caractérisée en ce que** ladite mutéine contient quatre sites de N-glycosylation choisis parmi le groupe constitué par les positions d'acide aminé Pro4, Arg23, Lys70 et Asp77.

5. Mutéine de l'interféron alpha humain recombinant selon la revendication 1, **caractérisée en ce que** ladite mutéine est stable en présence de N-glycanase à au moins 70% de sa concentration initiale, après 300 minutes.

6. Gène codant une mutéine de l'interféron alpha humain recombinant, dans lequel ledit gène code une séquence d'acides aminés ayant au moins une substitution d'acide aminé, donnant comme résultat la séquence consensus Asn-Xaa-Ser/Thr où le résidu Asn peut être N-glycosylé, dans la position Lys 70 avec un résidu Asn (Lys70Asn).

7. Gène codant une mutéine de l'interféron alpha humain recombinant selon la revendication 6, **caractérisée en ce que** ledit gène code une séquence d'acides aminés ayant un site de glycosylation dans la position Lys70 et en outre un site de N-glycosylation dans une position choisie parmi le groupe constitué par les positions Pro4, Arg23 et Asp77.

8. Gène codant une mutéine de l'interféron alpha humain recombinant selon la revendication 6, dans lequel ledit gène code une séquence d'acides aminés ayant trois sites de N-glycosylation, un site de N-glycosylation dans la position Lys70, et deux sites de N-glycosylation choisis parmi le groupe constitué par les positions Pro4, Arg23 et Asp77.

9. Gène codant une mutéine de l'interféron alpha humain recombinant selon la revendication 6, dans lequel ledit gène code une séquence d'acides aminés ayant quatre sites de N-glycosylation choisis parmi le groupe constitué par les positions d'acide aminé Pro4, Arg23, Lys70 et Asp77.

10. Formulation pharmaceutique comprenant au moins une mutéine de l'interféron alpha humain recombinant selon la revendication 1.

11. Formulation pharmaceutique comprenant au moins une mutéine de l'interféron alpha humain recombinant selon la revendication 2.

12. Formulation pharmaceutique comprenant au moins une mutéine de l'interféron alpha humain recombinant selon la revendication 3.

13. Formulation pharmaceutique comprenant au moins une mutéine de l'interféron alpha humain recombinant selon la revendication 4.

14. Formulation pharmaceutique comprenant au moins une mutéine de l'interféron alpha humain recombinant selon la revendication 5.

15. Formulation pharmaceutique selon les revendications 10 à 14, comprenant en outre des excipients pharmaceutiquement acceptables.

16. Formulation pharmaceutique selon les revendications 10 à 14, dans laquelle ladite formulation se présente sous forme de solution liquide, solide, émulsion, crème, gel et particules.

**17.** Utilisation de la mutéine selon l'une quelconque des revendications 1 à 6 pour préparer un médicament pour le traitement d'une maladie susceptible d'être traitée avec l'interféron alpha 2b, dans laquelle ladite maladie est choisie parmi le groupe constitué par mélanome, hépatite C chronique, hépatite B aigüe et chronique, hépatite non-A, non-B aigüe et chronique, sarcome de Kaposi, sclérose en plaques, verrues génitales, leucémie, infections virales.

**18.** Utilisation selon la revendication 17, dans laquelle ledit médicament est administré par une voie d'administration choisie parmi le groupe constitué par les voies sous-cutanée, parentérale, orale, sublinguale, intra-nasale, topique.

Signal peptide

```
 -2                    IFNalfaF                    Met  Ala  Leu  Thr   Phe  Ala   Leu   Leu  Val  Ala Leu
  1    TAACGAATTC  ACATCTACAA  TGGCCTTGAC  CTTTGCTTTA  CTGGTGGCCC
 -2    Leu Leu Val Leu  Ser Cys Lys  Ser Ser Cys  Ser  Val Gly    Cys  Asp  Leu  Pro
 51    TCCTGGTGCT  CAGCTGCAAG  TCAAGCTGCT  CTGTGGGCTG  TGATCTCCCT
 -2    Gln  Thr  His  Ser  Leu  Gly  Ser  Arg  Arg  Thr  Leu  Met Leu  Leu  Ala Gln Met
101    CAAACCCACA  GCCTGGGTAG  CAGGAGGACC  TTGATGCTCC  TGGCACAGAT
 -2   Met Arg  Arg   Ile   Ser  Leu  Phe  Ser  Cys  Leu  Lys  Asp Arg  His  Asp Phe Gly Phe
151    GAGCAGAATC  TCTCTTTTCT  CCTGCTTGAA  GGACAGACAT  GACTTTGGAT
 -2   Phe Pro Gln  Glu  Glu  Phe  Gly  Asn Gln  Phe  Gln  Lys Ala  Glu  Thr  Ile  Pro
201    TTCCCCAGGA  GGAGTTTGGC  AACCAGTTCC  AAAAGGCTGA  AACCATCCCT
 -2    Val Leu  His  Glu  Met  Ile  Gln  Gln  Ile  Phe  Asn Leu Phe  Ser  Thr  Lys Asp
251    GTCCTCCATG  AGATGATCCA  GCAGATCTTC  AATCTCTTCA  GCACAAAGGA
 -2   Asp Ser  Ser  Ala  Ala  Trp  Asp  Glu  Thr  Leu  Leu  Asp Lys  Phe  Tyr  Thr Glu Leu
301    CTCATCTGCT  GCTTGGGATG  AGACCCTCCT  AGACAAATTC  TACACTGAAC
 -2   Leu Tyr  Gln  Gln  Leu  Asn Asp  Leu  Glu  Ala  Cys  Val Ile  Gln  Gly  Val Gly
351    TCTACCAGCA  GCTGAATGAC  CTGGAAGCCT  GTGTGATACA  GGGGGTGGGG
 -2    Val  Thr  Glu  Thr  Pro  Leu  Met  Lys  Glu  Asp  Ser  Ile  Leu  Ala  Val  Arg Lys
401    GTGACAGAGA  CTCCCCTGAT  GAAGGAGGAC  TCCATTCTGG  CTGTGAGGAA
 -2   Lys Tyr  Phe  Gln  Arg  Ile  Thr  Leu  Tyr  Leu  Lys  Glu Lys  Lys  Tyr  Ser Pro Cys
451    ATACTTCCAA  AGAATCACTC  TCTATCTGAA  AGAGAAGAAA  TACAGCCCTT
 -2   Cys Ala  Trp  Glu  Val  Val  Arg  Ala Glu  Ile  Met  Arg Ser  Phe  Ser  Leu Ser
501    GTGCCTGGGA  GGTTGTCAGA  GCAGAAATCA  TGAGATCTTT  TTCTTTGTCA
 -2    Thr  Asn Leu  Gln  Glu  Ser  Leu  Arg  Ser  Lys  Glu  ***
551    ACAAACTTGC  AAGAAAGTTT  AAGAAGTAAG  GAATGAAAAC  TGGTTCAACA
601    TGGAAATGAT  TTTCATTGAT  TCGTATGCCA  GCTCACCTTT  TTATGATCTG
651    CCATTTCAAA  GACTCTAGAC  TAT
                    IFNalfaR
```

*Fig. 1*

**Fig. 2**

**Fig. 3**

*Fig. 4*

Fig. 5

Fig. 6 (A and B)

**Fig. 7**

**Fig. 8**

*Fig. 9*

## EP 2 048 158 B1

### REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9813381 A
- US 6620916 B
- US 5643564 A
- US 4184917 A
- WO 9621468 A
- US 20040180054 A
- US 6524570 B
- US 4179337 A
- US 5981709 A
- US 20060029573 A
- US 5738846 A
- WO 2005074892 A
- US 20040002474 A

- WO 9201055 A
- US 20050019871 A
- WO 03031464 A
- WO 9425615 A
- WO 9216640 A
- US 20030040037 A
- US 20030003529 A
- US 20020137134 A
- US 20020019342 A
- US 20030124645 A
- US 20020160460 A
- US 20020142370 A
- US 20020119516 A

**Non-patent literature cited in the description**

- **ISAACS ; LINDERMANN.** *Proc R Soc Lond Biol Sci,* September 1957, vol. 147 (927), 258-67
- **KOTENKO, S. V.** IFN-lambda mediate antiviral protection through .a distinct class II cytokine receptor complex. *Nat. Immunol.,* 2003, vol. 4, 69-77
- **SHEPPARD, P.** IL-28, IL-29 and their class II cytokine receptor IL-28R. *Nat. Immunol.,* 2003, vol. 4, 63-68
- **RADHAKRISHNAN et al.** Zinc-mediated dimer of human interferon - alpha 2b revealed by X-ray crystallography. *Structure,* 1996, vol. 4 (12), 1453-1463
- **KLAUS et al.** The three-dimentional high resolution structure of human interferon - alpha 2a determined by heteronuclear NMR spectroscopy in solution. *J. Mol. Biol.,* 1997, vol. 274, 661-675

- **MERIGAN T.** Induction of circulating interferon by synthetic anionic polymers of known composition. *Nature,* 1967, vol. 214, 416-417
- **A LAMER.** Interferon signal transduction. *Biotherapy,* 01 January 1996, vol. 8 (3-4), 175-81
- **RICHARD GRIEVE.** Cost-effectiveness of interferon or peg-interferon with ribavirin for histologically mild chronic hepatitis C. *Gut,* June 2005
- **GARY L. DAVIS.** *Interferon-alpha-2b alone or with ribavirin for the treatment of relapse of chronic hepatitis C,* 1998, vol. 339
- **RADHAKRISHNAN et al.** Zinc-mediated dimer of human interferon - alpha2b revealed by X-ray crystallography. *Structure,* 1996, vol. 4 (12), 1453-1463